# EUROPEAN PATENT APPLICATION

(11) **EP 1 239 038 A1**
(43) Date of publication of application: **11.09.2002**
(21) Application number: 01870038.5
(22) Date of filing: 07.03.2001
(51) Int. Cl.: C12N 15/12, C12N 15/10, C12Q 1/68, C07K 14/82, C07K 16/18, A61K 38/17, A61K 39/395, A61K 48/00, G01N 33/53, G01N 33/68, A01K 67/027

(54) **High-throughput identification of modulators of e2f activity**

(71) Applicant: Galapagos Genomics B.V., 2301 CA Leiden (NL)
(72) Inventor: The designation of the inventor has not yet been filed
(74) Representative: Prins, Hendrik Willem

(57) **Abstract**

The invention relates to the field of molecular genetics and medicine. In particular the present invention relates to the field of functional genomics. The present invention provides the methods and means for the identification of nucleic acid and the polypeptides encoded by these nucleic acids that have a function related to the E2F pathway, which were isolated in a high-throughput screening assay using the E2F transcription factor activity as a read-out. The identified compounds are suitable drug-targets to treat human diseases.

## Description

### Field of Invention

The invention relates to the field of molecular genetics and medicine. In particular the present invention relates to the field of functional genomics, more in particular to newly identified polynucleotides and the polypeptides encoded by these polynucleotides.

### Background of the invention

With the recent publication of the draft sequence of the human genome, we are facing the challenge to attribute functions to the estimated 30.000-40.000 human genes. Of particular interest is the identification of the gene products that function in human disease pathways, as these potential new drug targets may lead to novel therapeutic strategies. However, the study of gene functions in vertebrates is hampered by the multicellular nature and the complexity of the genome. Because of this, only a limited set of genes is currently available for the screening of drug compounds that interfere with their function. Therefore, there is a need for a method that allows for direct measurement of function of a single gene from a collection of genes in a high-throughput setting in appropriate *in vitro* assays. For this, the current knowledge of human disease pathways will need to be implemented in the development of cellular assays, to allow cell-based high-throughput functional screening methods. The present invention provides the methods and means for the identification of nucleic acid compounds that have a function related to the E2F pathway, which were isolated in a high-throughput screening assay using the E2F transcription factor activity as a read-out.

### The E2F transcription factors

One of the hallmarks of human cancer is the deregulation of the pRb tumor-suppressor pathway, either by mutation of pRb, its upstream regulator p16^{INK4a}, or by overexpression of cyclin D, which phosphorylates and thereby inactivates pRb (Weinberg, 1995). Besides the involvement of Rb in human cancers such as retinoblastoma and osteosarcoma, deregulation of the pRb pathway also underlies other human proliferative disorders such as the vascular disorders atherosclerosis and restenosis (Dzau et al., 1996; Ishizaki et al., 1996). In either case, deregulation of the pRb pathway will result in the activation of the downstream components of the pathway: the E2F transcription factors.

The relevance of E2F transcription factors in the regulation of cell proliferation is underscored by the observation that overexpression of E2F-1 in transgenic mice predisposes to tumorigenesis (Pierce et al., 1998). However, E2F-1 deficient mice develop a broad spectrum of tumors, suggesting that E2F may act as either an oncogene or a tumor suppressor, depending on the context in which activity is analysed (Yamasaki et al., 1996). In cell culture experiments, E2F-1 acts as a potent oncogene in transformation assays (Johnson et al., 1994; Singh et al., 1994). Furthermore, ectopic expression of E2F-1 is sufficient to drive quiescent cells into cell cycle (Johnson et al., 1993).

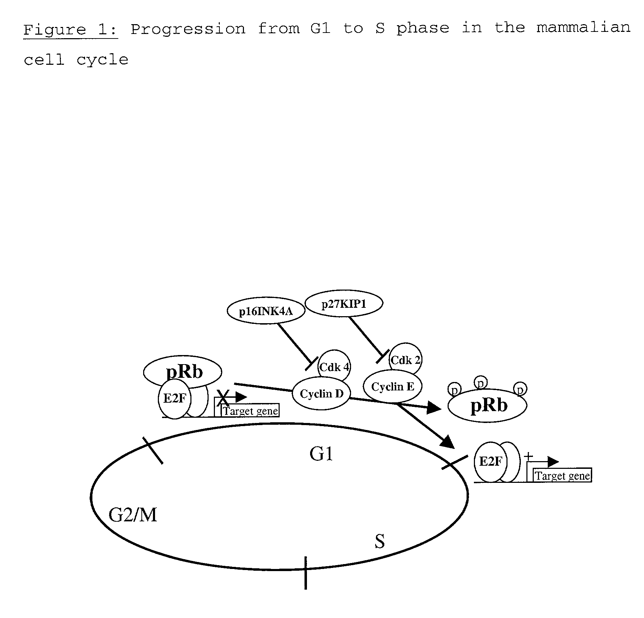

The E2F transcription factors are heterodimers containing a subunit encoded by the E2F gene family and a subunit encoded by the DP family of genes. To date six E2F genes (E2F-1 through 6) and two DP genes (DP-1 and DP-2) have been found in mammalian cells. E2F and DP proteins contain highly conserved DNA-binding and dimerization domains (Helin, 1998). The carboxy-terminal portion of E2F1-5 contain a potent transactivation domain but no equivalent activity has been found in E2F-6 nor in DP proteins. The different E2F heterodimers are regulated by interactions with members of the retinoblastoma gene family (pRb, p107 and p130). E2F1-3/DP complexes bind to pRb, E2F-4/DP heterodimers interact with pRb and p107, and E2F-5 is preferentially bound by p130. The association of E2Fs with pRb family members as well as their relative importance varies with specific stages of the cell cycle (Dyson, 1998). In general, p130/E2F complexes are primarily found in quiescent or differentiated cells and p107/E2F complexes are most prevalent in S phase cells. pRb/E2F complexes can be found in quiescent or differentiated cells, but are most evident as cells progress from G1 into S phase. The progression through the mammalian cell cycle is cooperatively regulated by several classes of cyclin-dependent kinases (Cdk) and their regulatory subunits: the cyclins (reviewed in (Sherr, 1994). The cyclins display a cyclic appearance as cells move from quiescence (G0) into the first gap phase (G1), through initiation of DNA synthesis (S) and via the second gap phase (G2) to mitosis (M). The activity of Cdk complexes depends on their expression levels, association with cyclins, phosphorylation status and the association with specific Cdk-inhibitors (CKIs). The CKIs can be divided into two classes based on their structures and targets. The first class involves the INK4a family including p16 ^{INK4a}, p15 ^{INK4b}, p18 ^{INK4c} and p19 ^{INK4d} that act as inhibitors of D-type cyclins by inhibiting their catalytic partners Cdk4 and Cdk6 (Hannon and Beach, 1994; Serrano et al., 1993). The second class consists of the Cip/Kip proteins p21 ^{Cip1}, p27 ^{Kip1} and p57 ^{Kip2} whose actions regulate cyclin D-, cyclin E- and A-dependent kinases, by binding to both the cyclin and Cdk subunits (Harper et al., 1993; Polyak et al., 1994). When quiescent cells enter the cell cycle, activated cyclin D-dependent kinases trigger the phosphorylation of the retinoblastoma tumor-suppressor protein Rb, and the family members p107 and p130 (Beijersbergen and Bernards, 1996; Xiao et al., 1996). Once pRb is primed with phosphates, Rb is further phosphorylated by cyclin E/Cdk2 complexes in late G1 phase (Lundberg and Weinberg, 1998). The phosphorylation of the Rb family members results in the release and activation of the E2F/DP transcription factors which play a central role in the control of cell proliferation. Inactivation of Rb, and subsequent activation of the E2F transcription factors at the G1/S boundery irreversibly commits the cells to complete the division cycle (See Figure 1 for schematic representation of G1 to S transition in the mammalian cell cycle).

Relatively little is known about the specific properties of the individual E2Fs but it is widely anticipated that different E2F heterodimers regulate various subsets of E2F target genes. E2F complexes bind to specific binding sites in the promoter regions of a number of cellular genes involved in DNA synthesis and regulation of the cell cycle, including DNA polymerase-α, *dhfr,* thymidine kinase, MCM genes, *orc1, cdk2, cdc2, cdc6,* cyclin A, cyclin E, *c-myc* and *b-myb* (reviewed in (Muller and Helin, 2000). There appear to be three generic types of E2F complexes: activator E2F complexes, in which the E2F activation domain promotes transcription; inhibited E2F complexes, in which the activation domain is masked by pRb-family proteins to give a complex that is essentially inert; and repressor E2F complexes, in which Rb-family proteins that are recruited to the DNA by E2F, assemble a repressor activity. Apparently, the activation of E2F target genes may either result from transcriptional activation or loss of active repression on the promoter regions. The mechanism of E2F-mediated transcriptional activation remains unresolved. Possibly, E2F can regulate transcription via the recruitment of either TBP or CBP to E2F regulated promoters (Hagemeier et al., 1993; Trouche et al., 1996). Also for Rb/E2F mediated repression the mechanism is unclear, but a role for both HDACs and the SWI/SNF nucleosome-remodeling complexes has been suggested (Luo et al., 1998; Trouche et al., 1997). Thus, E2F binding sites serve to repress as well as to activate cellular promoters, depending on the nature of the E2F complexes found in the cell.

As uncontrolled cell proliferation underlies many different human diseases, disrupting the deregulated pathways may provide a good strategy to treat these proliferative disorders. Indeed, recent studies suggest that interfering with the INK4a /cyclinD/pRb/E2F pathway may prevent uncontrolled proliferation. For example, *in* *vivo* tumor suppression was observed in breast xenografts subsequent to the treatment of established tumors with an adenoviral vector expressing the pRb protein (Demers et al., 1998). Furthermore, adenoviral mediated gene transfer of the retinoblastoma family proteins in a rat carotid artery model demonstrated that the inhibition of E2F activity resulted in reduced smooth muscle cell proliferation and prevented restenosis after angioplasty (Claudio et al., 1999). Also, it was shown with *in vivo* adenoviral gene therapy that directed overexpression of the p16 gene efficiently inhibited the pathology in an animal model of rheumatoid arthritis (Taniguchi et al., 1999). Moreover, *ex-vivo* gene therapy of human bypass grafts with E2F decoy oligodeoxynucleotides demonstrated that inhibition of E2F-mediated cell proliferation in these vein grafts lowered the failure rates of human primary bypass vein grafting (Mann et al., 1999).

Since the activation of E2F-dependent transcription is linked with cell transformation and proliferation, the events leading to elevated E2F activity are of great interest for developing new therapeutic strategies. Several types of regulation have been proposed to contribute to the activation process. The best studied mechanism of E2F activation is the disruption of preexisting pRb /E2F complexes by phosphorylation of the Rb family members, resulting in the release of 'free' and thereby active E2F, as described above (Beijersbergen and Bernards, 1996) (Figure 1). In addition, phosphorylation of E2F itself may disrupt pRb/E2F complexes (Fagan et al., 1994; Peeper et al., 1995). Also, both the phosphorylation and acetylation of E2F have been reported to regulate E2Fs transactivation potential (Martinez-Balbas et al., 2000; Marzio et al., 2000; Morris et al., 2000) Moreover, changing the subcellular localization of E2F complexes, which has been observed for E2F-4 containing complexes, may be a mechanism for regulating E2F activity (Muller et al., 1997; Verona et al., 1997). Furthermore, both E2F synthesis rates as ubiquitin-directed degradation will determine the amount of 'free' E2F in the cell (Hateboer et al., 1996; Hsiao et al., 1994; Sears et al., 1997). Although pRb is the best known regulator of E2F activity, the relative importance of the various suggested types of E2F regulation must be determined and new regulators may be identified. Clearly, those gene products that can alter E2F function are potential drug targets for proliferative disorders with deregulated E2F activity. However, since for most of the 40.000 genes a function still needs to be identified, there is a major hurdle to be taken to find those genes that act in the E2F pathway.

It is therefore an object of the present invention to provide a high-throughput, quantifiable, sensitive assay to screen for nucleic acid products that can modulate E2F activity. For this, a cell line was generated that could be used to screen for both activators and repressors of E2F activity. The introduction of nucleic acid libraries and the measurement of reporter activity was completely automated and provides the first demonstration of measuring E2F activity in a high-throughput fashion.

It is another object of the present invention to disclose besides the proof of concept, the identities of some of the validated hits from these screens. Those hits consist of both positive and negative modulators of E2F activity.

### Summary of the invention

In some particularly useful, but non-limiting aspects, the present invention provides functional genomics, compounds and medicines. Generally, it is an object of the invention to provide the identity of nucleic acids that are involved in human disease related cellular pathways. One specific object of the invention provides nucleic acids whose products are suitable drug-targets to treat human diseases.

One of the pathways frequently involved in human diseases such as cancer, is the E2F pathway. E2F transcriptional activity can be measured with the use of reporter constructs that contain multiple E2F-binding sites linked to a marker gene.

In order to efficiently isolate candidate nucleic acids that can upregulate or downregulate E2F reporter activity, nucleic acid libraries were screened for those nucleic acids that could alter E2F reporter activity in a high-throughput setting.

For this, an arrayed nucleic acid library together with an E2F reporter cell line was generated. The E2F reporter cell line was transduced with said nucleic acid library and the magnitude of activation or repression of the E2F reporter was measured in an automated fashion.

The present invention relates to polynucleotides and the encoded polypeptides that were identified in the high-throughput E2F reporter screen. Moreover, the present invention relates to vectors, host cells, antibodies and diagnostic methods for detecting diseases involving the discovered polynucleotides, and therapeutic methods for treating such diseases. The invention further relates to methods and means for drug compound screens designed to develop new therapeutic strategies.

### Detailed description of the invention

The present invention makes use of a method for identifying a group of nucleic acids from which the individual members comprise a nucleic acid that modifies the activity of cellular E2F, the method comprising: (i) generation of an arrayed nucleic acid library; (ii) generation of an E2F reporter cell line; (iii) transducing said E2F reporter cell line with said nucleic acid library; (iv) measuring the magnitude of activation or repression of expression of the E2F reporter in primary and secondary screens, wherein the secondary screen is similar to the primary screen and defined as rescreen; (v) validation of hits that were marked as activators or repressors in the primary screen and rescreen.

In a preferred embodiment, the present invention provides a method for identifying a sample nucleic acid that modulates E2F activity, said method comprising at least the following steps:
a) introduction of sample nucleic acid into a reporter cell line containing at least 2 E2F binding sites, preferably 4 and even more preferred 6 E2F binding sites, operably linked to a heterologous reporter gene,
b) assaying the resulting reporter gene expression levels, wherein induction or reduction of expression of the heterologous reporter gene indicates that the sample nucleic acid is a modulator of E2F activity,
c) isolation of the sample nucleic acid that caused a modulation of expression of the reporter gene as measured in step b,
d) rescreening said nucleic acids of step c, said rescreening comprising the steps of reintroducing said sample nucleic acid in the reporter cell line, and measuring modulation of expression of said reporter gene,
e) validating those nucleic acids obtained from step d that caused a modulation of reporter gene expression, said validation comprising the steps of introduction of said nucleic acid into a cell line together with a transient E2F reporter and measurement of the fold induction or reduction of expression of said transient reporter gene.

According to a further embodiment of the invention, the method of the present invention is being characterized in that at least step a and b are performed in a miniaturized high-throughput format.

According to a further embodiment of the present invention, said sample nucleic acid of step c causes an induction of expression, said induction being at least the background signal obtained in the absence of a nucleic acid plus three times the standard deviation on the measurement of said background signal.

In a further embodiment of the present invention, said sample nucleic acid of step c causes a repression of expression, said repression being at least a two-fold repression of reporter expression compared to background reporter expression levels.

It will be clear that with the "background signal obtained in the absence of a nucleic acid" and "background reporter expression levels" the same is intended, i.e. the signal generated by an empty vector. In this respect, the term "empty vector" relates to a vector in which no sample nucleic acid is cloned.

The sample nucleic acids according to the present invention can be genomic DNA, cDNA, previously cloned DNA, genes, ESTs, synthetic oligonucleotides, randomised sequences, antisense nucleic acids, genetic suppressor elements, ribozymes, mutant zinc fingers, antibody sequences or any combination thereof. The sample nucleic acid may encode a full-length protein, but it might also encode a partial, not full-length, protein.

The vector used for expressing the sample nucleic acid according to the present invention can be a vector for high level expression in *E. coli,* a yeast shuttle vector, or a yeast two-hybrid vector, a plant vector, an insect vector, a mammalian expression vector, including but not limited to, a herpes virus vector, a baculovirus vector, a lentivirus vector, a retrovirus vector, an alphavirus vector, an adenoviral vector or any combination thereof. Preferably, the sample nucleic acid is part of a set or library of sample nucleic acids that are cloned into a vector, whereby the set or library of sample nucleic acids may comprise between two and 2x10⁷ individual sample nucleic acids, and usually will contain between 100 and 1x10⁶ different sample nucleic acids. Explained in the examples is the generation of an adenoviral library in which cDNAs are cloned that originate from human placental tissue.

The introduction of a reporter construct as defined above, and to be used in a method according to the present invention, into a desired cell line can be performed by any transfection method known to the person skilled in the art, including but not limited to electroporation, calcium phosphate coprecipitation, liposomes and microinjection.

Although cells or cell lines used in a method according to the present invention could in principle be transiently transformed, stably-transformed cells or cell lines are preferred. Stable transformation of a cell line can, for example, be accomplished by using standard methods to co-transfect the cells with the vector of interest together with a second vector, which confers resistance to a selection agent such as an antibiotic. Alternatively, transformation can be carried out with a single vector containing both the promoter/reporter gene construct and the selection marker gene. For example, a viral vector may be used for such a single reporter vector approach. Selection of the cell clone transfected with the reporter gene can be performed by any drugmarker for which the transfected constructs confer resistance including, but not limited to, zeocin, puromycin, neomycin or hygromycin. In the example below, co-transfection was carried out using plasmid pBABE-puro (Morgenstern and Land, 1990), a plasmid that provides a dominant selectable marker for resistance to the drug puromycin in mammalian cells.

The reporter cell line to be used in a method according to the present invention as defined above can be generated in any desired cell that can be cultured *in vitro.* These cells can be obtained from any organism including, but not limited to, mammals such as humans, reptiles, amphibia, fish, nematodes, yeast, fungi, bacteria and plants. In the example below, the human osteosarcoma cell line U2OS was used.

Heterologous reporter genes which encode enzymes, antigens or other biologically active proteins, which can be monitored easily by biochemical techniques are preferred. Examples of suitable reporter genes include, but are not limited to, *E. coli Lac*Z, thymidine kinase, firefly and *Renilla* luciferase, secreted placental alkaline phosphatase, chloramphenicol acetyltransferase (CAT) and genes encoding fluorescent proteins such as eGFP. Expression products of the reporter genes can be measured using standard methods. The method will be obvious for a person skilled in the art and may include, for example, fluorescence measurements, enzyme and immunoassays. More than one copy may be utilized to increase the amount of reporter gene product. Although not usually required or desirable, it is also possible to include more than one type of reporter gene in the same cell. As described in the examples section, the luciferase gene was used as a reporter gene and a constitutive active *Renilla* luciferase expression vector was co-introduced in the cells together with the reporter vector, as a tool to determine relative cell quantities.

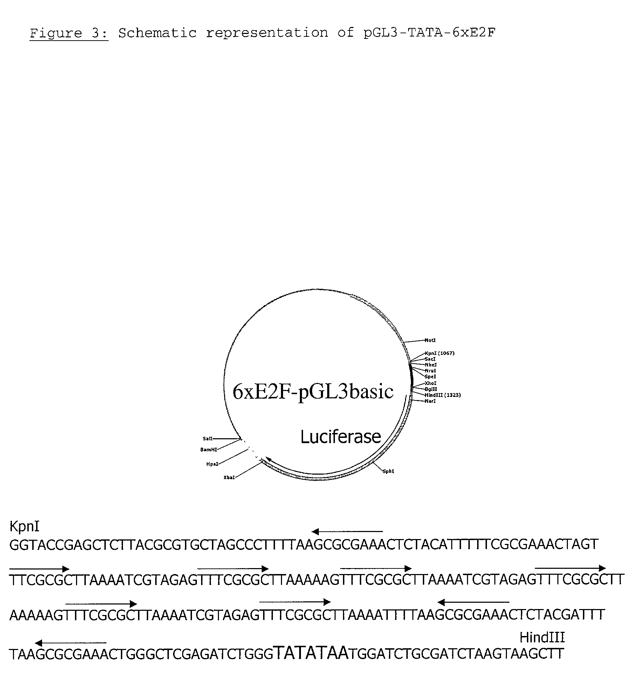

For a sensitive read-out of E2F activity with the use of a reporter construct the reporter should at least contain two E2F binding elements (E2F consensus site: 5'-TTT(C/G) (C/G)CGC-3') and preferably more, for example 4 or 6, or even more E2F binding sites. Explained in the example is the use of a reporter construct that contains at least 6 E2F binding elements. (pGL3-TATA-6xE2F, Figure 3). This reporter containing six E2F binding sites fused to the luciferase marker gene has yielded a sensitive tool to measure E2F activity in various experimental setups (Lukas et al., 1997). The 6xE2F-luciferase reporter construct is easily activated by overexpression of E2F, but can also be activated by endogenous E2F. The basal activity of this 6xE2F-luciferase reporter in cell culture assays will allow both the measurement of transactivation (induction of expression) and active repression (reduction of expression) on the reporter. The cloning of this reporter has not been described previously. Therefore, the part of the reporter containing the TATA elements, E2F sites, linker and transcription start site of the luciferase gene was sequenced, and part of the sequence is depicted in Figure 3.

The generation of the recombinant adenoviral vector library containing the sample nucleic acids, and the production of active recombinant viruses by introducing the viral vectors -containing the sample nucleic acid- in their respective packaging cell lines such as the PER.C6 or PER.C6/E2A packaging cell lines (US5994128, WO99/64582), are all performed in a high-throughput setting basically as described in WO99/64582 and further outlined in the examples. To identify and assign function to the product(s)encoded by the sample nucleic acids, the host reportercell line is transduced in a high-throughput setting with the recombinant adenoviral vectors that express the product(s) of the sample nucleic acids.

Furthermore, measurement of the function of the sample nucleic acids, which in the setup of the present invention is defined as a certain alteration of the reporter gene expression, is performed in a high-throughput setup. High-throughput is defined as handling between 100 and 100.000 samples per week and usually between 1000 and 10.000 samples per week.

In a further aspect, the invention provides a method to validate whether the group of candidate nucleic acids from the primary screen and rescreen are *bona fide* regulators of E2F reporter activity in a thirdscreen, defined as validation step, said validation method comprising:
(i) transient transfection of a cell line with reporter constructs which contain E2F responsive elements or control reporter constructs that contain no E2F elements,
(ii) transduction of said candidate nucleic acid vector in a transfected cell line, and,
(iii) measuring reporter activity to test whether modulation of E2F reporter is dependent on the presence of E2F elements and independent of the integrated reporter context.

In a preferred embodiment, the invention provides any nucleic acid identifiable by the methods according to the present invention.

The invention further provides the sequence identities of the group of nucleic acids that modulate E2F reporter activity in both primary screen, rescreen and validation experiments.

In a preferred embodiment, the present invention provides an isolated nucleic acid comprising a member selected from a group of nucleic acids identifiable as modulators of E2F reporter activity according to the described method, the group consisting of:
a) nucleic acid comprising a DNA sequence as given in SEQ ID NO 1 or 3. or the complement thereof,
b) nucleic acid comprising the RNA sequences corresponding to SEQ ID NO 1 or 3, or the complement thereof,
c) nucleic acid specifically hybridizing to the nucleotide sequence as defined in (a) or (b),
d) nucleic acid having a nucleotide sequence at least 65% identical to the sequence defined in (a),
e) nucleic acid encoding a protein with an amino acid sequence which is at least 65% identical to the amino acid sequence as given in SEQ ID NO 2 or 4,
f) nucleic acid encoding a protein comprising the amino acid sequence as given in any of SEQ ID NO 2 or 4,
g) nucleic acid which is degenerated as a result of the genetic code to a nucleotide sequence of a nucleic acid as given in SEQ ID NO 1 or 3, or as defined in (a) to (f),
h) nucleic acid which is diverged due to differences in codon usage between organisms to a nucleotide sequence encoding a protein as given in SEQ ID NO 2 or 4 or as defined in (a) to (g),
i) nucleic acid which is diverged due to the differences between alleles encoding a protein as given in SEQ ID NO 2 or 4, or as defined in (a) to (h),
j) nucleic acid encoding an immunologically active and/or functional fragment of a protein encoded by a DNA sequence as given in SEQ ID NO 1 or 3,
k) nucleic acid encoding a gene family member of the nucleic acid as given in SEQ ID NO 1 or 3, and,
1) nucleic acid encoding a protein as defined in SEQ ID NO 2 or 4 or a nucleic acid as defined in any one of (a) to (k) characterized in that said sequence is DNA, cDNA, genomic DNA or synthetic DNA.

In a preferred embodiment, the invention provides a nucleic acid molecule of at least 15 nucleotides in length specifically hybridizing with any of the nucleic acids of the present invention. In particular, longer nucleic acid molecules are contemplated, i.e. of about 20, 25, 30, 40, 50, 75, 100, 200 or even more nucleotides. It is to be understood that also shorter probes may be useful (having for instance 10, 11, 12, 13 or 14 nucleotides). Different types of hybridization techniques and formats are well known in the art. The said nucleic acid molecule may be labeled with, for example, a radioactive isotope, thereby allowing the detection of the hybrid. As such, the present invention provides methods for detecting the nucleic acids of the present invention.

In a further embodiment, the invention provides a nucleic acid molecule of at least 15 nucleotides in length as described above, wherein said nucleic acid molecule is liable to act as a primer for specifically amplifying a nucleic acid of the present invention, or a part thereof. It is to be understood that said primers can be shorter, e.g. 10, 11, 12, 13, or 14 nucleotides, or longer, e.g. 16, 17, 18, 19, 20, 25, or 30 nucleotides.

Sets of said primers may be used in any well described amplification technique known in the art such as Polymerase Chain Reaction (PCR) or NASBA techniques, thereby allowing the amplification and subsequent detection of the nucleic acid of the present invention. Preferably said primers may also be used to specifically amplify the nucleic acids of the present invention. As such, the present invention provides methods for detecting the nucleic acids of the present invention.

The present invention is also directed to variants of the nucleotide sequence of the nucleic acid disclosed in SEQ ID NOs 1 or 3, or the complementary strand thereto. "Variants" refers to a nucleotide sequence differing from the nucleotide sequence from the nucleic acid of the present invention, but retaining essential properties thereof. Generally, variants are overall closely similar, and, in many regions, identical to the nucleotide sequences of the present invention.

The present invention is also directed to nucleic acid molecules which comprise, or alternatively consist of, a nucleotide sequence which is at least 65%, 70%, 75%, 80%, 85%, 90%, 95%, 96%, 97%, 97,5%, 98%, 98,5%, 99% or 99,5% identical to the nucleotide sequences as represented in SEQ ID NOs 1 or 3 or the complementary strand thereto, or parts thereof. Said parts are preferaly unique parts.

By a nucleic acid having a nucleotide sequence at least, for example, 95% "identity" to a reference nucleotide sequence of the present invention, it is intended that the nucleotide sequence of said nucleic acid is identical to the reference sequence except that the nucleotide sequence may include up to five point mutations per each 100 nucleotides of the reference nucleotide sequence. In other words, to obtain a nucleic acid having a nucleotide sequence of at least 95% identity to a reference nucleotide sequence, up to 5% of the nucleotides in the reference sequence may be deleted or substituted with another nucleotide, or a number of nucleotides up to 5% of the total nucleotides in the reference sequence may be inserted into the reference sequence. As a practical matter, whether any particular nucleic acid molecule is at least 65%, 70%, 75%, 80%, 85%, 90%, 95%, 96%, 97%, 97,5%, 98%, 98,5%, 99% or 99,5% identical to a nucleotide sequence of the present invention can be determined using known algorithms. A preferred method for determining the best overall match between a query sequence (a sequence of the present invention) and a subject sequence can be determined using a Blast search (Altschul et al., 1997).

Nucleic acids which specifically hybridise to any of the strands of the nucleic acid molecules of the present invention as specified under SEQ ID NO 1 or 3.under stringent hybridisation conditions or lower stringency conditions are also particularly encompassed by the present invention. "Stringent hybridisation conditions" refers to an overnight incubation at 68°C in a solution comprising 5xSSC (750 mM NaCl, 75 mM trisodium citrate), 50 mM sodium phosphate (pH 7.6), 5x Denhardt's solution, 10% dextran sulfate and 20 µg/ml denatured sheared salmon sperm DNA, followed by washing the filters in 0.1xSSC at about 65°C. Changes in the stringency of hybridisation are primarily accomplished through the manipulation of the SSC dilution in the washing steps (higher concentration SSC in washing buffer results in lower stringency) and the temperature (lower washing temperature results in lower stringency). For example, lower stringency conditions include washes performed at 1xSSC and at 55-60°C. Hybridisation under high and low stringency conditions are principles which are well understood by the person skilled in the art (see for instance Sambrook et al. Molecular Cloning: A laboratory manual. Cold Spring Harbor laboratory press 1989).

Methods which are well known to those skilled in the art may be used to construct expression vectors containing at least a fragment of the nucleic acids of the present invention together with appropriate transcriptional and translational control elements. These methods include in vitro recombinant DNA techniques, synthetic techniques, and *in vivo* genetic recombination. Such techniques are described, for example, in Sambrook et al. Molecular Cloning: A laboratory manual. Cold Spring Harbor laboratory press 1989.

The invention provides a vector, including but not limited to, a vector for high level expression in *E. coli,* a yeast shuttle vector, or a yeast two-hybrid vector, a plant vector, an insect vector, a mammalian expression vector, including but not limited to, a herpes virus vector, a baculovirus vector, a lentivirus vector, a retrovirus vector, an alphavirus vector, an adenoviral vector or any combination thereof.

In a preferred embodiment, the invention provides a vector comprising a nucleic acid sequence of the present invention. As such, said nucleic acid is a member selected from a group of nucleic acids identifiable as modulators of E2F reporter activity.

In a preferred embodiment said vector is an expression vector wherein the nucleotide sequence is operably linked to one or more control sequences allowing the expression of said sequence in prokaryotic and/or eukaryotic host cells.

In a preferred embodiment said vector is an adenoviral vector.

In a preferred embodiment said vector is generated from an adenoviral adapter vector which contains the left ITR and part of the E2B region, and in which the E1 region has been exchanged for a mammalian promotor, a polylinker sequence, and a polyadenylation signal.

As will be understood by those skilled in the art, it may be advantageous to produce products encoded by the before mentioned isolated nucleotide sequences possessing non-naturally occurring codons. For example, codons preferred by a particular prokaryotic or eukaryotic host can be selected to increase the rate of protein expression or to produce an RNA transcript having desirable properties, such as a longer half-life, thereby increasing the amount of expressable polypeptides in a cell, which may be desirable for multiple applications.

The nucleotide sequences of the present invention can be engineered using methods generally known in the art in order to alter protein encoding sequences for a variety of reasons, including but not limited to, alterations which modify the cloning, processing, and/or expression of the gene product. DNA shuffling by random fragmentation and PCR reassembly of gene fragments and synthetic oligonucleotides may be used to engineer the nucleotide sequences. For example, site-directed mutagenesis may be used to insert new restriction sites, alter glycosylation patterns, change codon preference, produce splice variants, introduce mutations, and so forth.

Furthermore, natural, modified, or recombinant nucleotide sequences may be ligated to partial or complete nucleic acid sequences of the present invention to encode a fusion protein. For example, to screen peptide libraries for inhibitors of the product of the nucleic acids of the present invention, it may be useful to encode a chimeric protein that can be recognized by a commercially available antibody. A fusion protein may also be engineered to contain a cleavage site located between the protein coding sequence and the heterologous protein sequence, so that the protein may be cleaved and purified away from the heterologous moiety.

In a further embodiment, the present invention provides a polynucleotide sequence encoding the antisense nucleic acid of the invention. Such antisense nucleic acids can be constructed by recombinant DNA technology methods standard in the art.

In a preferred embodiment, the present invention provides a vector comprising an antisense nucleic acid. In a more preferred embodiment, said vector is an expression vector wherein the antisense polynucleotide sequence is operably linked to one or more control sequences allowing the expression of said sequence in prokaryotic and/or eukaryotic host cells.

Antisense technology can be used to control gene expression, for example for inhibition of gene expression as described in the art. As such, antisense nucleic acids can be used as antagonist compounds, and may be employed to regulate cell growth and proliferation effects both *in vitro* and *in vivo* of the polypeptides of the present invention.

Potential antagonists according to the invention also include catalytic RNA, or a ribozyme. Ribozymes cleave mRNA at site-specific recognition sequences and can be used to destroy mRNAs corresponding to the polynucleotides of the present invention. The construction and production of ribozymes is well known in the art. As in the antisense approach, ribozymes of the invention can be used as antagonist compounds, and can be delivered to cells to, for example, inhibit *in vitro* or *in vivo* cell growth and proliferation effects of the polypeptides of the present invention.

The invention further provides the nucleic acids sequences for controlling gene expression with the use of a dsRNA approach. It has been described in the art (WO 99/32169) that providing dsRNA to a target cell can result in the downregulation of the translation/expression of any desired RNA sequence that may be present in said cell. As such, the nucleic acids of the present invention can be used as antagonist compounds, and may be employed to regulate cell growth and proliferation effects both *in vitro* and *in vivo* of the polypeptides of the present invention.

In a further embodiment, the invention provides a host cell containing an integrated or episomal copy of any of the nucleotide sequences of the present invention or any functional parts thereof. In a more preferred embodiment, the invention provides a host cell containing a vector comprising a nucleic acid sequence according to the present invention. Said host cell is obtained from any organism including, but not limited to, mammals such as humans, amphibia, reptiles, fish, nematodes, yeast, fungi, bacteria, insects and plants. In this regard, the term "functional parts" refers to any part of the nucleotide sequence of the present invention which exhibits substantially a similar, but not necessarily identical, activity as the complete nucleotide sequence, i.e. is able to modulate E2F function as measured in the method of the invention, with or without dose dependency.

In a preferred embodiment, the invention provides an isolated polypeptide encodable by any of the before mentioned nucleic acids, or a variant or a derivative thereof, or an immunologically active and/or functional fragment thereof.

"Variants" of a protein of the invention are those peptides, oligopeptides, polypeptides, proteins and enzymes which contain amino acid substitutions, deletions and/or additions relative to the said protein with respect to which they are a homologue, without altering one or more of its functional properties, in particular without reducing the activity of the resulting variant. In other words, the term "variant" refers to a polypeptide or protein differing from the polypeptide or protein of the present invention, but retaining essential properties thereof. Generally, variants are overall closely similar, and, in many regions, identical to the polypeptide or protein of the present invention. For example, a homologue of said protein will consist of a bioactive amino acid sequence variant of said protein. To produce such homologues, amino acids present in the said protein can be replaced by other amino acids having similar properties, for example hydrophobicity, hydrophilicity, hydrophobic moment, antigenicity, propensity to form or break α-helical structures or β-sheet structures, and so on. Amino acid substitutions are typically of single residues, but may be clustered depending upon functional constraints placed upon the polypeptide; insertions will usually be of the order of about 1-10 amino acid residues and deletions will range from about 1-20 residues. Preferably, amino acid substitutions will comprise conservative amino acid substitutions.

Insertional amino acid sequence variants of a protein of the invention are those in which one or more amino acid residues are introduced into a predetermined site in said protein. Insertions can comprise amino-terminal and/or carboxy-terminal fusions as well as intra-sequence insertions of single or multiple amino acids. Generally, insertions within the amino acid sequence will be smaller than amino or carboxyl terminal fusions, of the order of about 1 to 10 residues. Deletional variants of a protein of the invention are characterised by the removal of one or more amino acids from the amino acid sequence of said protein.

Amino acid variants of a protein of the invention may readily be made using peptide synthetic techniques well known in the art, such as solid phase peptide synthesis and the like, or by recombinant DNA manipulations. The manipulation of DNA sequences to produce variant proteins which manifest as substitutional, insertional or deletional variants are well known in the art.

"Derivatives" of a protein of the invention are those peptides, oligopeptides, polypeptides, proteins and enzymes which comprise at least about 5 contiguous amino acid residues of said polypeptide but which retain the biological activity of said protein. Preferably said derivatives will comprise at least 6, 7, 8, 9, 10, 11, 12, 13, 14, or 15 contiguous amino acid residues of said protein. A "derivative" may further comprise additional naturally-occurring, altered glycosylated, acylated or non-naturally occurring amino acid residues compared to the amino acid sequence of a naturally-occurring form of said polypeptide. Alternatively or in addition, a derivative may comprise one or more non-amino acid substituents compared to the amino acid sequence of a naturally-occurring form of said polypeptide, for example a reporter molecule or other ligand, covalently or non-covalently bound to the amino acid sequence such as, for example, a reporter molecule which is bound thereto to facilitate its detection.

With "immunologically active" is meant that a molecule or specific fragments thereof such as epitopes or haptens are recognized by, i.e. bind to antibodies.

In the context of the current invention are embodied homologues, derivatives and/or immunologically active fragments of any of the new E2F modulating sequences as defined above.

The term "homologue" relates to the molecule in a non-human species, that corresponds to the molecule of the present invention, i.e. able to modulate E2F function as measured in the method of the invention, with or without dose dependency.

In a preferred embodiment, the invention provides a method for producing said polypeptide, the method comprising culturing said host cells of the invention as defined above under conditions allowing the expression of the polypeptide and recovering the produced polypeptide from the culture. Alternative methods for producing said polypeptides of the invention are well known.

In a more preferred embodiment, the invention provides a polypeptide having an amino acid sequence as given in SEQ ID NO 2 or 4, or a variant or a derivative thereof, or an immunologically active and/or functional fragment thereof.

The present invention is also directed to polypeptides, which comprise, or alternatively consist of, an amino acid sequence which is at least 65%, 70%, 75%, 80%, 85%, 90%, 95%, 96%, 97%, 97,5%, 98%, 98,5%, 99% or 99,5% identical to the amino acid sequences of the present invention, wherein said amino acid sequence of the invention, the so-called reference sequence, is at least 30 amino acids in length. However, for reference sequences smaller than 30 amino acids the polypeptide must consist of an amino acid sequence which is at least 65%, 70%, 75%, 80%, 85%, 90%, 95%, 96%, 97%, 97,5%, 98%, 98,5%, 99% or 99,5% identical to the reference sequence.

By a polypeptide having an amino acid sequence of at least, for example, 95% "identity" to a reference amino acid sequence of the present invention, it is intended that the amino acid sequence of the polypeptide is identical to the reference sequence except that the amino acid sequence may include up to five amino acid alterations per each 100 amino acids of the reference polypeptide amino acid sequence. In other words, to obtain a polypeptide having a amino acid sequence at least 95% identical to a reference amino acid sequence, up to 5% of the amino acids in the reference sequence may be deleted or substituted with another amino acid, or a number of amino acids up to 5% of the total amino acids in the reference sequence may be inserted into the reference sequence. As a practical matter, whether any particular polypeptide is at least 65%,70%, 75%, 80%, 85%, 90%, 95%, 96%, 97%, 97,5%, 98%, 98,5%, 99% or 99,5% identical to a polypeptide sequence of the present invention can be determined using known algorithms. A preferred method for determining the best overall match between a query sequence (a sequence of the present invention) and a subject sequence can be determined using BLASTp (Altschul et al., 1997).

In a preferred embodiment, the invention provides an antibody specifically recognizing the polypeptides of the present invention, or a specific epitope of said polypeptide. The term epitope refers to portions of a polypeptide having antigenic or immunogenic activity in an animal, preferably a mammal, and most preferably in a human. Epitope-bearing polypeptides of the present invention may be used to induce antibodies according to methods well known in the art including, but not limited to, *in vivo* immunization, *in vitro* immunization, phage display methods or ribosome display.

In specific embodiments, antibodies of the present invention cross-react with murine, rat and/or rabbit homologues of human proteins and the corresponding epitopes thereof. Further included in the present invention are antibodies which bind polypeptides encoded by nucleic acids which hybridise to a polynucleotide of the present invention under stringent hybridisation conditions (as described herein). As such, the present invention provides a method for detecting the polypeptides of the present invention, the method comprising the use of the antibodies in immunoassays for qualitatively or quantitatively measuring levels of the polypeptides of the present invention in biological samples.

Antibodies of the present invention may act as agonists or antagonists of the polypeptides of the present invention. Preferably, antibodies of the present invention bind an antigenic epitope as disclosed herein, or a particular portion of the proteins of the present invention.

Antibodies of the present invention may be used, for example, but not limited to, to purify, detect, target, and inhibit the activity of the polypeptides of the present invention, including both *in vitro* en *in vivo* diagnostic and therapeutic methods.

In a particular aspect, the invention provides a pharmaceutical composition comprising a substantially purified nucleic acid, polypeptide or antibody according to the present invention, possibly in conjuction with a suitable carrier. Suitable carriers for adding to the nucleic acids, polypeptides or antibodies of the present invention are well known in the art.

Besides the involvement of E2F in cell proliferation, deregulation of the E2F pathway can also induce apoptosis under certain conditions. Numerous studies have reported that the inhibition of E2F activity can prevent apoptosis in cultured cells. Therefore, the nucleic acids, polypeptides and antibodies identifiable as modulators of E2F activity according to the present invention as mentioned above, can be used to provide methods and means to treat both proliferative as well as apoptosis-related disorders or diseases.

In one preferred embodiment, the present invention provides nucleic acids and fragments thereof, that can be used to regulate cellular proliferation and/or apoptosis in a desired target cell, *in vitro* or *in vivo.*

In a preferred embodiment, the invention provides a method for regulating cell proliferation and/or apoptosis, the method comprising introduction of the expression vectors comprising the sample nucleic acids of the present invention in a desired target cell, *in vitro* or *in vivo.*

In a preferred embodiment, the invention provides polypeptides, including protein fusions, or fragments thereof, for regulating cell proliferation and apoptosis in a desired target cell, *in vitro* or *in vivo.* For example, inhibition of aberrant cell division may occur as a direct result of administering polypeptides to mammalian, preferably human, cells. Delivering compositions containing the polypeptide of the invention to targeted cells, may occur via association via heterologous polypeptides, heterologous nucleic acids, toxins, or prodrugs via hydrophobic, hydrophilic, ionic and/or covalent interactions.

In another embodiment, the present invention provides antibody-based therapies for regulating cell proliferation or apoptosis in a desired target cell, *in vitro* or *in vivo.* Antibody-based therapies involve administering of anti-polypeptide and anti-polynucleotide antibodies to a mammalian, preferably human, cell. Methods for producing anti-polypeptide and anti-polynucleotide antibodies are known in the art. Such antibodies may be provided in pharmaceutically acceptable compositions as known in the art.

In a preferred embodiment, the present invention provides the use of a nucleic acid encoding a protein, comprising an amino acid sequence which is at least 65% identical to SEQ ID NO 2, for repressing E2F activity

In a preferred embodiment, the present invention provides the use of a protein, comprising an amino acid sequence which is at least 65% identical to SEQ ID NO 2, for repressing E2F activity.

In a preferred embodiment, the present invention provides the use of a nucleic acid encoding a protein, comprising an amino acid sequence which is at least 65% identical to SEQ ID NO 4, for repressing E2F activity.

In a preferred embodiment, the present invention provides the use of a protein, comprising an amino acid sequence which is at least 65% identical to SEQ ID NO 4, for repressing E2F activity.

In a preferred embodiment, the present invention provides the use of a nucleic acid according to the invention for modulating E2F activity.

In a preferred embodiment, the present invention provides the nucleic acids, polypeptides or antibodies of the present invention for use as a medicament (both for treatment as for diagnosis of diseases). Said treatment according to the present invention refers to preventing, treating and/or alleviating diseases or disorders as defined above and below.

In an even more preferred embodiment, the present invention provides nucleic acids, polypeptides or antibodies of the present invention, for the preparation of a medicament for preventing, treating or alleviating diseases at the cellular level including, but not limited to, proliferative disorders and apoptosis-associated disorders.

By "proliferative disorders" is meant according to the present invention any human or animal disease or disorder, affecting any one or any combination of organs, cavities, or body parts, which is characterized by single or multiple local abnormal proliferation of cells, groups of cells, or tissues, whether benign or malignant. By "proliferative disorders" is also meant the induction of proliferation in cells, groups of cells, or tissues, whether or not it occurs *in vivo* or *ex vivo.* Examples of diseases that can be treated, prevented or diagnosed by nucleic acids, polypeptides or antibodies of the present invention include, but are not limited to, anaemia, lymphocytopenia, thrombopenia, and neutropenia. Also several treatments, like stem cell therapy, transplantation (e.g. of Langerhans cells), tissue repair (e.g. bone repair and bone replacement), and corrective surgery, might greatly benefit from an induction of proliferation in cells, groups of cells, or tissues.

Further examples of proliferative disorders or diseases that can be treated, prevented, and/or diagnosed by nucleic acids, polypeptides or antibodies of the present invention include, but are not limited to, various types of cancer such as retinoblastoma, osteosarcoma, adenocarcinoma, leukemia, lymphoma, melanoma, myeloma, sarcoma, and teratocarcinoma, cancers of the adrenal gland, bladder, bone, bone marrow, brain, breast, cervix, gall bladder, ganglia, gastrointestinal tract, heart, kidney, liver, lung, muscle, ovary, pancreas, parathyroid, penis, prostate, salivary glands, skin, spleen, testis, thymus, thyroid, and uterus, hyperplasias of the thyroid, endometrium, pituitary gland and adrenal gland, lipodystrophia, lymphoproliferative diseases, psoriasis and vascular disorders such as atheriosclerosis and restenosis, transplant-related myeloproliferative diseases, lymphocytosis and immunoproliferative diseases related to infection and autoimmune diseases, granulomatous diseases, like, for instance, histiocytosis and sarcoidosis, fibromatosis, multicentric histiocytosis, polycythaemia, and thrombocythaemia.

By "apoptosis-associated disorders" is meant any human or animal disease or disorder, affecting any one or any combination of organs, cavities, or body parts, which is characterized by abnormal cell survival or increased cell death.

Examples of apoptosis-associated disorders or diseases that can be treated, prevented, and/or diagnosed by nucleic acids, polypeptides or antibodies of the present invention include, but are not limited to, AIDS and other infectious or genetic immunodeficiencies, neurodegenerative diseases such as Alzheimer's disease, Parkinson's disease, amyotrophic lateral sclerosis, retinitis pigmentosa, and cerebellar degeneration, myelodysplastic syndromes such as aplastic anemia, ischemic injuries such as myocardial infarction, stroke, and reperfusion injury, toxin-induced diseases such as alcohol-induced liver damage, cirrhosis, and lathyrism, wasting diseases such as cachexia, viral infections such as those caused by hepatitis B and C, and osteoporosis.

In one preferred embodiment the present invention provides a gene therapy method for treating, alleviating or preventing disorders and diseases including, but not limited to, proliferative disorders and apoptosis-associated disorders. The gene therapy methods relate to the introduction of nucleic acid sequences into an animal to achieve expression of a polypeptide of the present invention. This method requires a nucleic acid, which codes for a polypeptide of the invention that is operatively linked to a promotor or any other genetic element necessary for the expression of the polypeptide in the target tissue. Such gene therapy and delivery techniques are known in the art, see, for example, EP0707071

In one embodiment, the nucleic acid of the invention is delivered as a naked polynucleotide. The term naked nucleic acid refers to sequences that are free from any delivery vehicle that acts to assist, promote or facilitate entry into a cell, including viral sequences, viral particles, liposome formulations, lipofectin or precipitating agents and the like. The naked nucleic acids can be delivered by any method known in the art, including, but not limited to, direct needle injection at the delivery site, intravenous injection, topical administration, catheder infusion, and so-called "gene guns".

In another embodiment, the nucleic acids may be delivered with delivery vehicles such as viral sequences, viral particles, liposome formulations, lipofectin or precipitating agents and the like. Viral vectors that can be used for gene therapy applications include, but are not limited to, a herpes virus vector, a baculovirus vector, a lentivirus vector, a retrovirus vector, an alphavirus vector, an adeno-associated virus vector or an adenoviral vector or any combination thereof. In a preferred embodiment, viral vectors used are replication deficient, for example such as described for adenoviral vectors in WO99/64582.

Delivery of the nucleic acids into a subject may be either direct, in which case the subject is directly exposed to the nucleic acid or nucleic acid-carrying vectors, or indirect, in which case, cells are first transformed with the nucleic acids *in vitro,* and then transplanted into the subject. These two approaches are known, respectively, as *in vivo* or *ex vivo* gene therapy and are well described.

In a more preferred embodiment, the present invention provides a gene therapy method for treating or alleviating cell based disorders including, but not limited to, proliferative disorders and/or apoptosis-associated disorders comprising the use of the vectors according to the present invention.

Cells into which nucleic acids or polypeptides of the present invention can be introduced, for example for therapeutic purposes, encompass any desired available cell type, including but not limited to epithelial cells, endothelial cells, keratinocytes, fibroblasts, muscle cells, hepatocytes, synoviocytes, adipocytes, bone cells, blood cells such as T lymphocytes, B lymphocytes, monocytes, macrophages, neutrophils, eosinophils, megakaryocytes, mast cells, granulocytes and various stem or progenitor cells, in particular hematopoietic stem or progenitor cells.

In a further aspect, the present invention provides nucleic acids or nucleic acid encoded products, and antibodies that can be used in drug compound screens directed to find drugs that can be used in the treatment of cellular diseases including, but not limited to, proliferative diseases and/or apoptosis-associated diseases.

In a preferred embodiment the invention provides a screening method, the method comprising contacting the polypeptides of the present invention, or binding fragments thereof, with a selected compound suspected having antagonist or agonist activity, and assaying the activity of these polypeptides following contacting the compound. The screening for therapeutic compounds may be any of a variety of drug screening techniques known in the art. The polypeptide or fragment employed in such a screening may be affixed to a solid support, expressed on a cell surface, free in solution, or located intracellularly.

The present invention also contemplates the use of competitive drug screening assays in which neutralizing antibodies capable of binding polypeptides of the present invention specifically compete with a test compound for binding to the polypeptides or fragments thereof. In this manner, the antibodies are used to detect the presence of any peptide that shares one or more antigenic epitopes with a polypeptide of the invention.

In a preferred embodiment, the invention provides a method for screening compounds for preventing, treating and/or alleviating proliferative disorders or apoptosis-associated disorders comprising the steps of:
a)contacting the compounds to be screened with a nucleic acid or polypeptide according to the present invention, and,
b)determining whether said compound effects an activity of said nucleic acid or said polypeptide

In another embodiment, the invention provides the compounds or products identified by the drug screening method according to the present invention.

In a preferred embodiment, the invention provides a method for the production of a composition comprising the steps of admixing a compound identifiable by a method of the invention with a pharmaceutically acceptable carrier.

The nucleotide sequences presented in the present invention may be extended utilizing a partial nucleotide sequence and employing various methods known in the art to detect the full sequence in case said sequence would only be a part of a coding region as well as upstream sequences such as promoters and regulatory elements. As such, the invention provides means and methods to regulate the expression of said nucleic acids by providing to a subject or host cell molecules that can positively or negatively influence said regulatory elements of said sequences identified in the present invention.

In a preferred embodiment, the invention provides a method for treating and/or alleviating proliferative disorders and/or apoptosis-associated disorders comprising the use of a molecule, which allows to interfere with the expression of a polynucleotide or polypeptide of the present invention in a patient in need of such a treatment.

In a preferred embodiment, the invention provides nucleic acids, polypeptides or antibodies, for the preparation of a diagnostic kit for detecting proliferative disorders or apoptosis-associated disorders.

In a preferred embodiment, the invention provides a kit for the diagnosis of proliferative disorders or apoptosis-associated disorders in a patient comprising a nucleic acid of the invention, a probe or primer according of the invention, a polypeptide of the invention, or an antibody of the invention, possibly in conjunction with suitable buffers, means for detection or detection format parts (such as, for example, solid carriers, e.g. membranes. Suitable formats and technologies for designing diagnostic kits on the basis of the above are well known in the art. Preferred formats include any type of microarray format known in the art.

In a further embodiment, the invention provides a method for diagnosing a pathological condition or a susceptibility to a pathological condition in a subject comprising the steps of:
a) determining the presence or absence of a mutation in the nucleic acid of the invention, including mutations in the genomic and regulatory sequences of said nucleic acid, in a biological sample, and,
b) diagnosing a pathological condition or a susceptibility to a pathological condition based on the presence or absence of said mutation.

In another embodiment, the invention provides a method for diagnosing a pathological condition or a susceptibility to a pathological condition in a subject comprising the steps of:
a) determining the presence or amount of the nucleic acid of the invention or expression of the polypeptide of the invention in a biological sample, and,
b) diagnosing a pathological condition or a susceptibility to a pathological condition based on the presence or amount of said nucleic acid or expression of said polypeptide.

Said diagnosis may be preferably achieved by means of detection using probes, primers or antibodies of the invention.

In a preferred embodiment, the invention provides a transgenic non-human animal comprising one or more copies of a nucleic acid of the present invention stably integrated in the genome, or an animal comprising regulatory elements that modulate the expression of a nucleic acid according to the present invention.

In a preferred embodiment, the invention provides a knock-out non-human animal comprising a deletion of one or two alleles encoding a nucleic acid of the present invention, or animal models comprising a targeted mutation in the genomic region, including regulatory sequences, comprising any of the nucleic acid sequences of the present invention.

In a preferred embodiment, the invention provides the use of a transgenic or knock-out non-human animal, according to the present invention, as a model system.

As the E2F activity status of a cell is tightly linked with its proliferation potential, the disruption of growth regulatory pathway that act parallel of the E2F pathway may also (indirectly) affect cellular E2F activity, for example via cell cycle 'sensors' that act on E2F via feedback mechanisms. Because of this, the modulators of E2F activity that are described in the present invention may affect cell growth by directly regulating E2F, but also by modulating other growth-regulating pathways such as the pathways regulated by the gene products of the genes including, but not limited to, *myc, ras,* NFκB, APC, TGF-β, *src, c-abl,* p53, *neu, myb, fos, jun, rel, raf, mos* and erbB. Therefore, the invention provides nucleic acids, polypeptides and antibodies that, upon administering to cells, can modulate growth regulatory pathways, either directly or indirectly via the E2F pathway.

The following figures and examples are meant to illustrate the embodiments of the present invention and are in no way to be construed as limiting the present invention.

### Short Description of the figures

### Figure 1:

Progression from G1 to S phase in the mammalian cell cycle.

### Figure 2:

Schematic representation of the construction of adenoviral Placenta library.

### Figure 3:

Schematic representation of pGL3-TATA-E2F-luc.

### Figure 4:

Schematic representation of pIPspAdapt8-L61Ras.

### Figure 5:

Schematic representation of pIpSpAdApt3-E2F2.

### Figure 6:

Schematic representation of pIpSpAdApt3-E2F3.

### Figure 7:

Schematic representation of pIpSpAdApt6-p16INK.

### Figure 8:

Schematic representation of pIpSpAdApt6-p27KIP.

### Figure 9:

Schematic representation of pIpSpAdApt6-EGFP.

### Figure 10:

Schematic representation of pCLIP-L61Ras.

### Figure 11:

Schematic representation of pIpSpAdApt6-LacZ.

### Figure 12:

Schematic representation of the various E2F reporter cell lines tested + controls.

### Figure 13:

Schematic representation of the optimalization infection conditions E2F-reporter cell line IC5. Assay at different MOI.

### Figure 14:

Schematic representation of the optimization of infection conditions E2F reporter cell line 1C5. Assay at 48 or 72 hours after infection.

### Figure 15:

Schematic representation of the optimization of infection conditions E2F reporter cell line 1C5. High/Low serum conditions.

### Figure 16:

Schematic representation of rescreen: reporter assay with first hits from 1500 screen.

### Figure 17:

Schematic representation validation (transient reporter) of hits from rescreen (1500).

### Figure 18:

Schematic representation of reporter assay in 384 wells format with control viruses from control virus plate.

### Figure 19:

Schematic representation of the performance of control viruses that were implemented in the 11.000 library virus reporter screen.

### Figure 20:

Schematic representation of the results obtained for 51 hits in the first screen and rescreen at approximate MOIs of 600 and 2000.

### Figure 21:

Comparison of the results of the hits obtained in first 11.000 screen and retested in rescreen.

### Figure 22:

Schematic representation validation (transient reporter) of hits from rescreen (11.000). A: E2F reporter, B: control reporter.

### Figure 23:

Nucleotide and deduced amino acid sequences of Hits H35 and H57.

### Examples

### Example 1. Library construction

An arrayed adenoviral human placenta cDNA library was constructed and screened using an E2F reporter assay. Under 'arrayed adenoviral cDNA library', we understand a collection of adenoviruses (contained in 96 well plates) mediating the expression of various (human) cDNAs, in which every well contains a single virus type. Further details about the concept of arrayed adenoviral libraries can be found in WO 99/64582 (Arrayed adenoviral libraries for performing functional Genomics).

### - Construction of the primary cDNA library

Construction of the primary cDNA library was subcontracted to Life Technologies. In brief, mRNA emanating from a 12 week old human placenta was used for the (oligo dT-primed) generation of the first strand cDNA using the Superscript II method (Life Technologies). After second strand synthesis, cDNAs were directionally cloned (*Sal*I*-Not*I) into the pIPspAdapt6 vector (described in WO 99-64582). The cDNA library was then transformed into *Escherichia coli* (DH10B). 5' sequencing analysis on 167 clones revealed that 98.8% of the plasmids from the library contained inserts and that 24% of the inserts were full length cDNAs.

### - Isolation and storage of individual cDNA clones

Part of the bacteria transformed with the primary cDNA library were plated onto an LB agar growth medium (+ 100µg/ml ampicillin) contained in Bio-assay dishes (Life Technologies). These bio-assay dishes were then incubated at 37°C for 18 hrs. Bacteria were plated at a density of 1500 cfu/plate, thereby allowing recognition and automatic picking of individual colonies by a QPix apparatus (Genetix). This device picked individual bacterial colonies and further inoculated 300 µl of liquid LB growth medium (+ 100 µg/ml ampicilin) in 96 well plates. Inoculation occurred in such a way that every single well of the 96 well plate was inoculated with bacteria emanating from a single colony. These 96 well plates were then incubated for 18 hrs in a rotary shaker (New Brunswick Scientific, Innova, floor model) at 37°C, 300 rpm. After this incubation period, bacterial cultures reached an OD (600 nm) of approximately 4. 100 µl of bacterial cultures were then mixed with 100 µl of 50% glycerol using a Multimek robot (Beckman Coulter) and stored at -80°C. These plates are defined as 'glycerol stock plates'.

### - Preparation of plasmid DNA

A second step in the construction of the adenoviral cDNA library was the 'arrayed' purification of DNA of individual plasmids from the primary cDNA library in amounts sufficient for adenovirus generation. For this purpose, a bacterial culture was prepared as follows. The glycerol stock plates were thawed and 3 µl of the bacterial culture was transferred to a 96 well plate filled with 280 µl of liquid LB growth medium (+ 100 µg/ml ampicillin) using a CybiWell robot (CyBio). These inoculated plates were then incubated for 18 hrs in a rotary shaker (37°C, 300rpm) (New Brunswick Scientific, Innova, floor model). This incubation step yielded bacterial cultures with an OD (600) of approximately 8. Centrifugation of the 96 well plates (3 min, 2700 rcf) was performed to pellet the bacteria. All centrifugations of 96 well plates were performed in an Eppendorf microtiterplate centrifuge (type 5810). The supernatant was then removed by decanting into a waste container. The lysis of bacterial cells and precipitation of proteins and genomic DNA was performed applying the classical alkaline lysis protocol. The (3) buffers used to perform alkaline lysis were purchased from Qiagen. In a first step, the bacterial pellet was resuspended into 60 µl of buffer P1. In a second step, 60 µl of buffer P2 was added to the resuspended bacterial cells and a mixing step and 5 min incubation time were applied to achieve complete cell lysis. Finally, 60 µl of buffer P3 was added and a mixing step applied for precipitation of proteins and genomic DNA. The 96 well plates were then centrifuged (40 min, 3220 rcf). The supernatant (100 µl) was collected and transferred to new V-bottom 96 well plates containing 80 µl of isopropanol (for precipitation of the plasmid DNA) using a CybiWell robot (CyBio). The plates containing the pellet were discarded. The 96 well plates were centrifuged (45 min, 2700 rcf) and the supernatant discarded by decanting in a waste container. To remove salt traces, the pellet was washed with 100 µl of 70% ethanol and the 96 well plates were centrifuged again (10 min, 2700 rcf). Supernatant was removed again by decanting in a waste container and the DNA pellets were allowed to dry for 1 h in a laminar air flow cabinet. Finally, the DNA was dissolved in 20 µl of sterile TE buffer (1 mM Tris, pH7.6+ 0.1mM EDTA). Plates containing the dissolved DNA (further defined as 'DNA plates') were stored at -20°C until further use.

### - DNA quantification

Before use for transfection of PerC6/E2A cells, the plasmid DNA preparations contained in 96 well plates were quantified. For this purpose, 5 µl of plasmid DNA was pipetted from the 'DNA plates' and transferred to a 96 well plate containing 100 µl of TE buffer. Then 100 µl of 'quantification solution' was added. This solution was prepared by dissolving 2 µl of SybrGreen (Molecular Probes) into 10 ml of TE Buffer. After a mixing step, measurement was performed in a Fluorimeter (Fluostar, BMG) with following settings: emission : 485 nm; excitation: 520 nm , gain: 35. A standard curve was generated by performing a measurement using different dilutions (in TE buffer) of a standard DNA sample (= lambda DNA). By fitting results for the individual DNA samples on this curve, DNA concentration per well was calculated. The mean DNA concentration per well for each 'DNA plate' was then calculated. On average, a DNA concentration of 20 ng/µl of DNA was obtained.

### - Transfection of PerC6/E2A cells

As mentioned in the description of the primary cDNA library construction, cDNAs produced from the placenta tissue were cloned into the pIPspAdApt6 plasmid. This adapter plasmid contains the 5' part (bp 1-454 and bp 3511-6093) of the adenovirus serotype 5 genome (in which the E1A gene was deleted and a CMV promoter, multiple cloning site and SV40-derived poly adenylation signal have been inserted). Two other materials needed for the generation of recombinant adenovirus particles were a cosmid ¹ and a packaging cell line ² (WO99/64582).
¹ The cosmid (pWE/Ad.AflII-rITRΔE2A) contained the main part of the adenovirus serotype 5 genome (bp 3534-35953) from which the E2A gene was deleted.
² The PerC6/E2A packaging cell line was derived from human embryonic retina cells (HER) transfected with plasmids mediating the expression of the E1 and E2A genes.

In order to obtain viruses, this adapter plasmid was cotransfected into a packaging cell line PerC6/E2A with the cosmid. Once the adapter and helper plasmids were transfected into the PerC6/E2A cell line, the complete Ad5 genome could be reconstituted by homologous recombination. The helper and adapter plasmids contained homologous sequences (bp 3535-6093), which were a substrate for this recombination event. The E1 and E2A gene products, which were required for adenoviral replication, were provided by the PerC6/E2A cell line *in trans.* The adenoviral genes integrated into the genome of the PerC6/E2A cell line and the reconstituted adenoviral genome share no homologous sequences, which renders the reversion to replication competent adenoviral particles virtually impossible.

The 'DNA plates' that were prepared and quantified as described above, were used for transfection of the PerC6/E2A cell line. Prior to this transfection, the plasmids contained in this plates were linearised by digestion with the PI-*PspI* restriction enzyme (New England Biolabs). For this purpose, a certain volume of plasmid DNA (representing 66.7 ng of DNA on average, as calculated from the average DNA concentration of each DNA plate) was pipetted from the 'DNA plates' into a V-bottom 96 well plate containing a restriction mix composed of 1X restriction buffer (New England Biolabs : 10mM Tris-HCl pH 8.6, 10mM MgCl2, 150 mM KCl, 1 mM DTT), 100µg/ml BSA and 6 units of PI-*PspI* restriction enzyme (from a stock of 20 U/µl). For each 'DNA plate', an identical volume of plasmid was used for all wells. Transfer of the DNA samples from the 'DNA plate' to the plate containing the restriction mix and subsequent mixing was performed with a JoBi Well robot (CyBio). The plates containing the restriction mix were then put in plastic boxes containing humidified paper towels (to avoid evaporation) and incubated at 65°C for 4 hrs. The helper plasmid (pWE/Ad.AflII-rITRΔE2A) (which was prepared in batch using the Qiagen Maxi-prep kits) was also linearised with the *Pac*I restriction enzyme (New England Biolabs).

The transfection of the PerC6/E2A cells with the linearised adapter and helper plasmids was set up as follows. 0.1867 µl of linearised helper plasmid (containing 93 ng of DNA) was mixed with 1.11 µl of serum free 2XDMEM (Life Technologies) to form a 'helper mix`. 0.597 µl of Lipofectamine (Life Technologies) was mixed to 1.11 µl of 2XDMEM to form a 'lipo mix'. In each well of 96 well plates containing the linearised adapter plasmids, 1.3µl of 'helper mix and 1.7 µl 'Lipo mix' were pipetted using a CyBi-Well robot (CyBio, equiped with a `dropper'). The plates were then incubated for approximately 1 hour at room temperature before addition of 28.5 µl of serum-free DMEM. Mixing was performed by pipetting up and down the mix three times (CyBi Well robot). Using the same device, 30 µl of the mix was transferred to 96 well plates containing PerC6/E2A cells seeded at a density of 2,25x10⁴ cells/well. Cells were seeded into 100 µl of PerC6/E2A medium (composed of DMEM (Life Technologies) containing 10% FBS (Life Technologies), 50 µg/ml gentamycin and 10 mM MgCl2), but prior to addition of the 30 µl of the DNA/Lipofectamine mix, the medium was removed from (all wells of) the plates. An incubation time of 3 hours at 39°C, 10% CO2 was then applied. 170 µl of PerC6/E2A medium was then added to the plates and an overnight incubation at 39°C, 10%CO2 applied. The 96 well plates containing the transfected PerC6/E2A cells were incubated at 34°C, 10% CO2 during 3 weeks. This temperature allowed the expression of the E2A factor, which was required for adenoviral replication. During this incubation time, viruses were generated and replicated, as revealed by the appearance of CPE (cytopathic effect). The percentage of the wells showing CPE was scored, which allowed the evaluation of the efficiency of virus production. Typically, 55% to 65% of all wells processed showed CPE at this stage. The 96 well plates were stored at -80°C until further propagation of the viruses.

### - Virus propagation

The final virus propagation step aimed at obtaining a higher percentage of wells showing CPE and more homogenous virus titers. Viruses were propagated according to following procedure. The transfection plates stored at -80°C were thawed at room temperature for about 1 hour. By means of a 96 channel Hydra dispenser (Robbins), 20 µl of the supernatant was transferred onto PerC6/E2A cells seeded in 96 well plates at a density of 2,25x10⁴ cells/well in 180 µl of DMEM + 10%FBS. After handling of series of 96 viruses, needles of the dispensor were desinfected and sterilised by pipetting up 60 µl of 5% bleach three times. The traces of bleach present in the needles were removed by 3 successive washes with 70 µl of sterile water. Cells were incubated at 34°C, 10% CO2 during approximately 10 days and the number of wells showing CPE was then scored. On average, the number of wells showing CPE increased by 10% as compared to the original scoring after transfection, which represented 65% to 75% of the total number of wells processed. The plates were then stored at -80°C until aliquotation.

From the 200 µl of crude cell lysate containing the library viruses after the propagation step, 6 aliquots of 25 µl were prepared in 384 well plates using a 96 channel Hydra dispensor. This implied that from 4 96 well plates, 6 identical 384 well aliquot plates were prepared. Desinfection of the needles in between the individual plates was achieved by a triple washing step with 200 µl 5% bleach and a triple washing step with 250 µl sterile water to remove bleach traces. The 384 well aliquot plates were then stored at -80°C until further use in the assays.

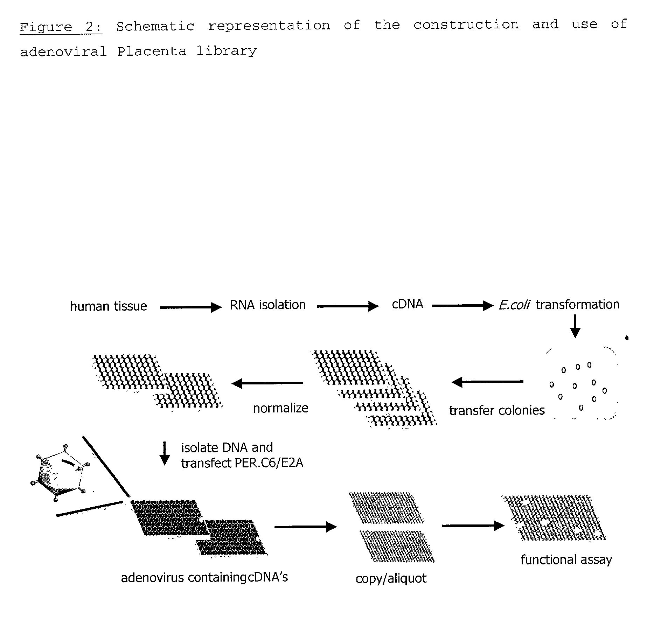

A schematic representation of the library construction is shown in figure 2.

### Example 2. Construction U2OS E2F reporter cell line

### Generation of Stable E2F-luciferase reporter in U2OS

Day 1: 4 x 10 cm dishes with 70% confluent U2OS cells were transfected with the calcium phosphate precipitation technique (van der Eb and Graham, 1980) according to the following transfection table:

| | #1 | #2 | #3 |
|---|---|---|---|
| pBABE-puro | 1 µg | 1 µg | 1 µg |
| 6xE2F-luc | 10 µg | 10 µg | 10 µg |
| CMV-renilla | 1 µg | 1 µg | 1 µg |
| CMV-E2F1 | - | 0.5 µg | 2.5 µg |

Day 2: Plates were washed twice in PBS and fresh medium was added. Cells are cultured in Dulbecco's modified eagle's medium containing 10% fetal calf serum (FBS) supplemented with penicillin/streptomycin.
Day 3: Cells were split 1:5, 1:50, 1:100, 1:200, 1:500.
Day 4: Medium was replaced with medium containing 1 µg/ml puromycin and was refreshed every third day.
Day 22: Medium was removed and the plates were incubated at 37°C for 4 minutes in PBS. Colonies were picked using a p200 pipette and transferred to a 24-wells plate containing medium with puromycin. 50 colonies from #1, 25 from #2 and 25 from #3 (transfection table) were isolated. Medium was refreshed every second day following day 22.
Day 36: 100 clones grown up from day 22 were split 1:4 and reseeded in 24 wells plates. Medium was changed every second day following this.
Day 42: 48 out of 50 clones from #1 were frozen for storage in liquid nitrogen, 2 were lost under selection.
Day 43: 24 of each #2 and #3 were frozen and stored in liquid nitrogen.
Day 42/43: One well of each clone was split in two and used for first round selection

All clones were tested in 24 well plates for induction of the luciferase reporter by E2F, and repression by p16Ink and p27Kip. Results were normalized for *Renilla* expression. From these initial experiments (data not shown), 5 cell lines were chosen that were further tested on 96 well plates.

### Example 3. Optimalization E2F assay in 96 well format

The 5 above mentioned stable U2OS-derived E2F-reporter cell lines (1C5; 1C31; 2C10; 3C1; and 3C20) were tested on 96 well plates. Viruses used were ΔE1/ΔE2A adenoviruses transducing E2F2; E2F3; p16INK; p27Kip *LacZ;* eGFP (all generated from pIPspAdApt plasmids); and empty virus (generated from pIPspAdApt 6).

Adenoviral constructs transducing E2F2 and E2F3 were created by digestion of the parental plasmids containing HA-E2F2 and HA-E2F3 cDNAs (Xu et al., 1995) with BamHI and HindIII, isolation of the inserts over an agarose gel, and ligation of the insert fragments in BamHI/HindIII-digested pIPspAdApt 3 (see WO99/64582), to generate pIPspAdApt3-E2F2 and pIPspAdApt3-E2F3, respectively (Figures 5 and 6).
Adenoviral constructs transducing p16INK and p27Kip were created by HindIII-XhoI digestion of the parental plasmids containing p16INK and p27-HA cDNAs (Beijersbergen et al., 1995; Peeper et al., 1997) and ligation of the isolated insert fragments in HindIII/SalI-digested pIPspAdApt6 (see WO99/64582), to generate pIPspAdApt6-p16INK and pIPspAdApt6-p27Kip, respectively (Figures 7 and 8).

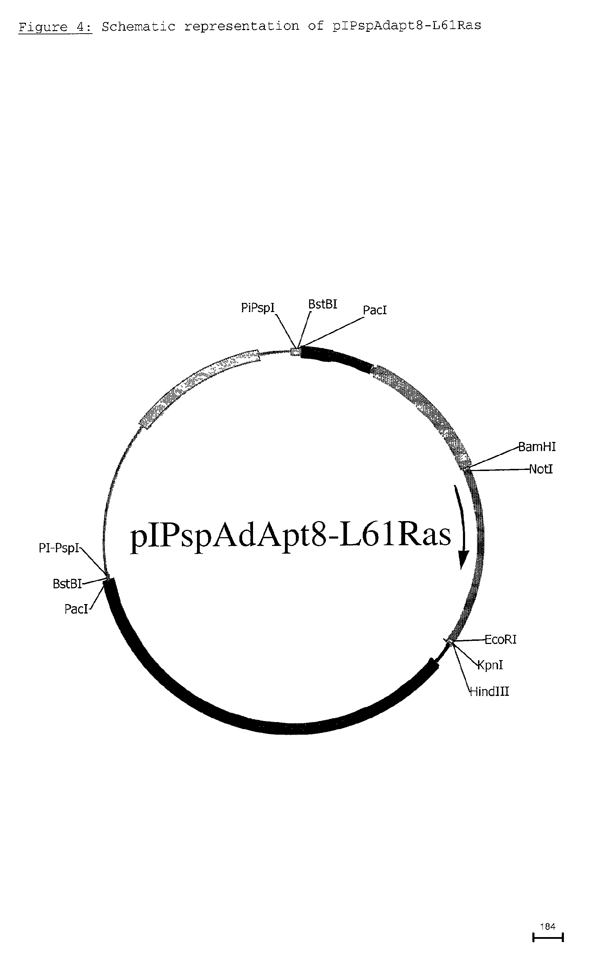

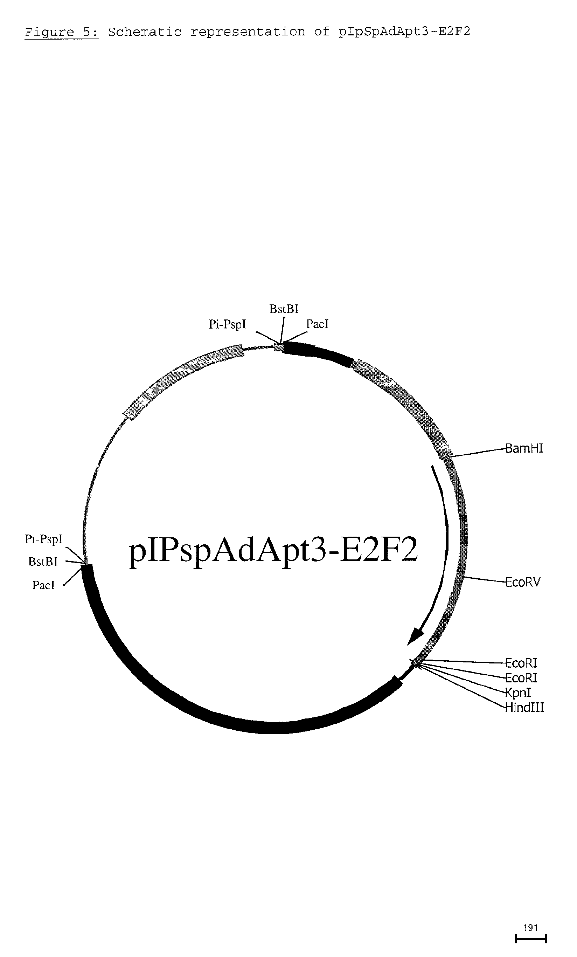

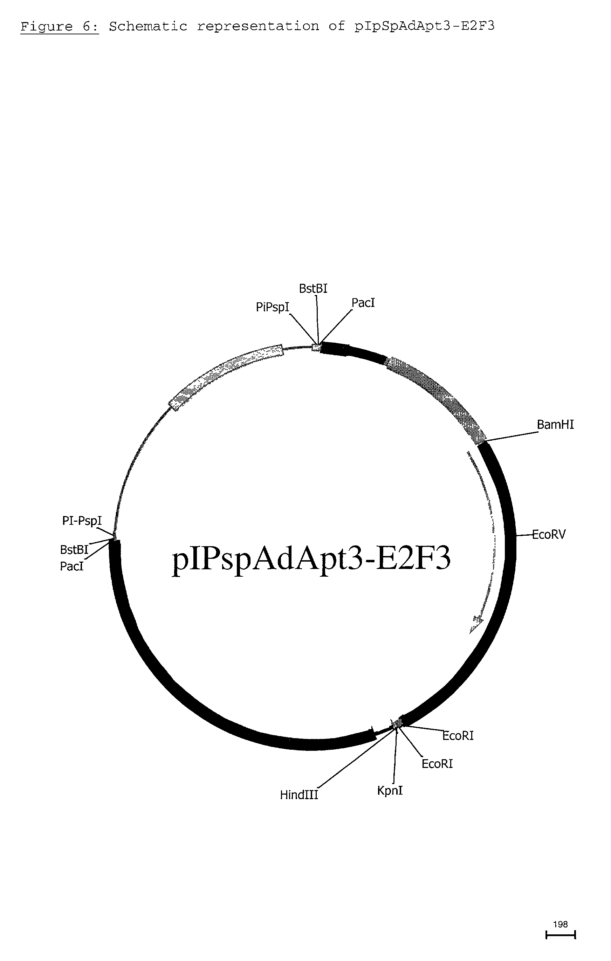

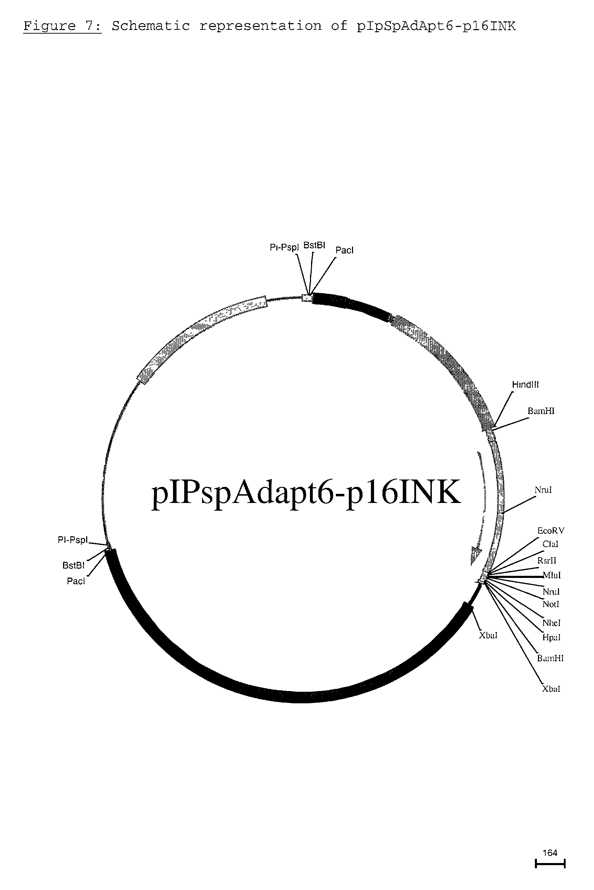

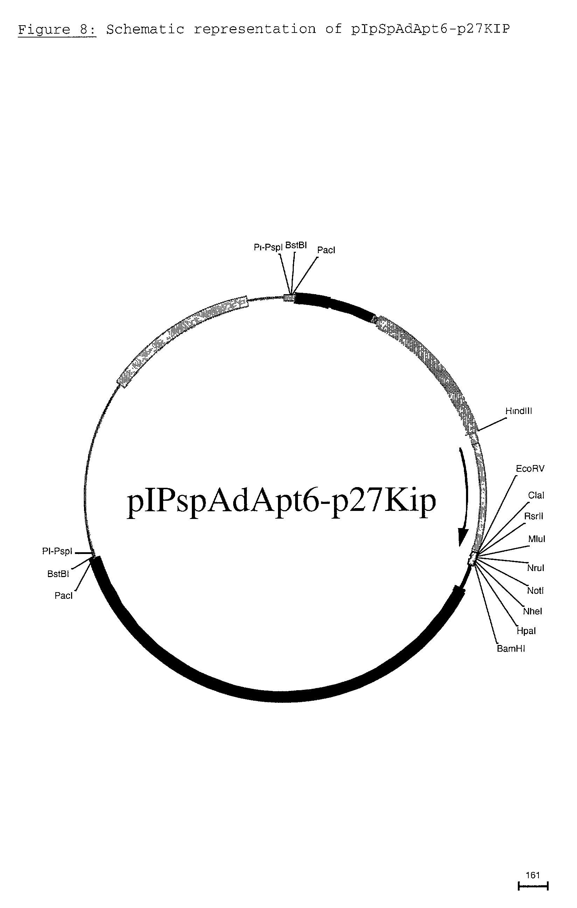

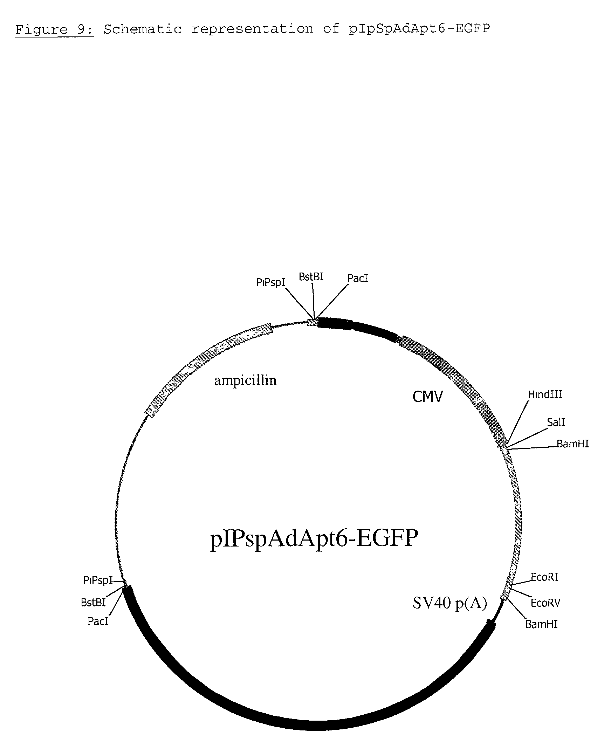

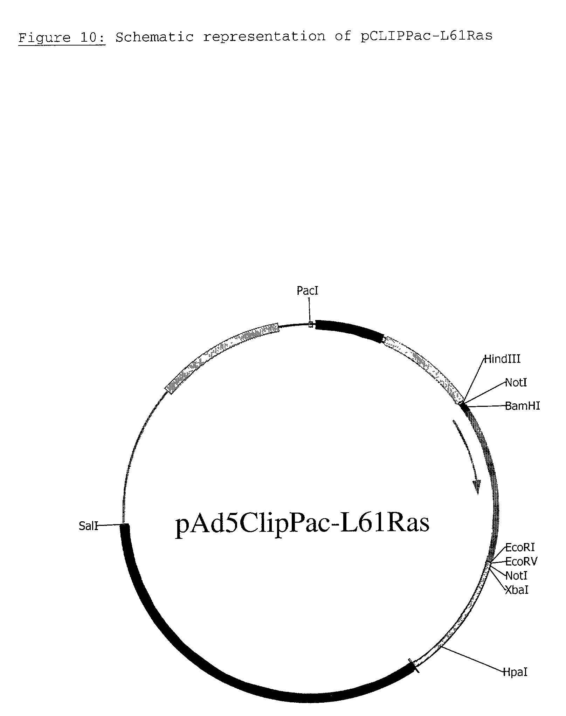

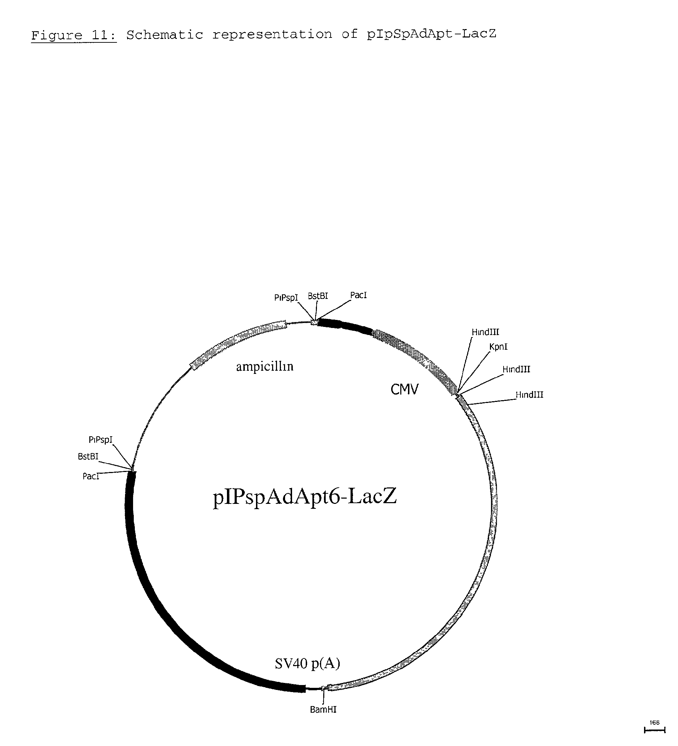

The adenoviral construct transducing L61Ras was created by digestion of the parental construct pMT2SM-L61Ras (Schaap et al., 1993) with SalI, blunting of the overhang with Klenow polymerase and dNTP's, and digestion with EcoRI. The isolated insert fragment was ligated in pAd5CLIPPac, which was digested with HindIII, blunted with Klenow polymerase and dNTP's, and redigested with EcoRI, resulting in pAd5ClipPac-L61Ras (Figure 10). The isolated insert fragment was also ligated in HpaI/EcoRI-digested pIPspAdApt 8, leading to pIPspAdApt8-L61Ras (Figure 4).
pIPspAdApt6-lacZ (Figure 11), was constructed by digestion of pIPspAdApt6 with KpnI and BamH1, followed by insertion of the correspondingly digested and purified nls-*lacZ* gene from pCLIP-lacZ (WO 00/52186).
pIPspAdApt6-EGFP, was constructed by releasing the EGFP insert by HindIII-EcoRI digestion from the plasmid pEGFP (Clontech; catalogus number 6077-1), followed by insertion into HindIII/EcoRI-digested pIPspAdApt6 to generate pIPspAdApt6-EGFP (Figure 9).

ΔE1/ΔE2A adenoviruses were generated from these adapter plasmids by co-transfection of the helper plasmid pWEAd5AflII-rITR.dE2A in PER.C6/E2A packaging cells, as described (WO99/64582).

The 5 E2F-luciferase reporter cell lines were seeded at 5x10³ cells per well in 96 well plates and incubated overnight at 37°C in a humidified incubator at 10% CO2 in 100 µl of DMEM supplemented with 10% heat inactivated FBS. The next day, cells were infected with control viruses, transducing p16Ink, p27Kip, E2F2, E2F3, eGFP, and Empty, at a known MOI of 100 in duplo.

24 hours after infection, the medium of the 96 well plates was replaced with 100 µl of fresh medium.

72 hours after infection, the medium was removed from the wells. The cells were washed once with Phosphate Buffered Saline and frozen at -20°C in 100 µl of PBS.

After thawing and resuspension of the cell lysate, 100 µl of Steady-Glo (Promega) was added and incubated for 15 minutes at room temperature. 100 µl of each well of the resulting mixture, was transferred to a Wallac Black&White sample plate and luciferase activity was determined on a Wallac Trilux 1450 microbeta Liquid Scintillation and Luminescence Counter.

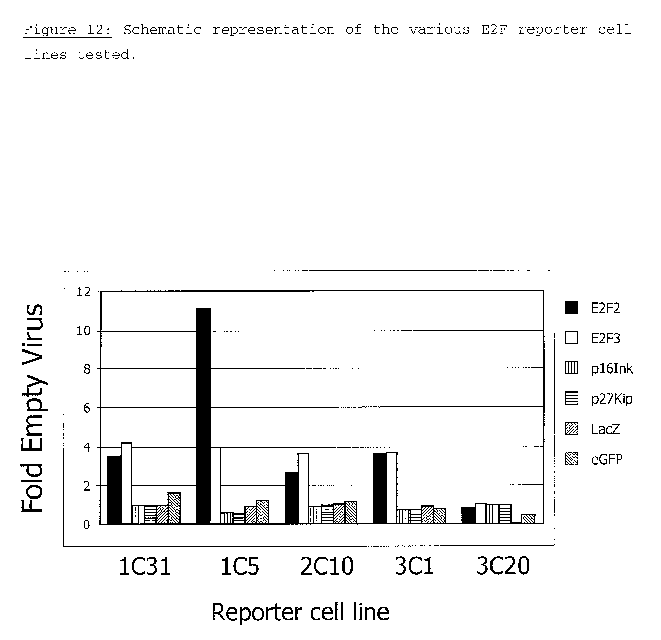

Results are expressed relative to the empty vector control for each cell line (see Figure 12). From these experiments, it was concluded that cell line 1C5 gave the best activation of the luciferase reporter after infection with E2F2- or E2F3-transducing viruses, while repression by pl6Ink or p27Kip could also be scored (see also Figure 12). Further experiments to optimise the set up of the assay were therefore performed with cell line 1C5.

To determine the optimal MOI for infection, 5x10³ U2OS-1C5 cells were seeded per well in a 96 well plate, using DMEM with 10% heat inactivated FBS and 1 µg/ml puromycin (Clontech) (hereonafter referred to as U2OS medium), and incubated overnight at 37°C in a humidified incubator at 10% CO2.

After 24 hours, cells were infected with adenoviruses transducing E2F2, E2F3, p16Ink, p27Kip, LacZ, eGFP and empty. MOI used were 20, 100 and 500. All experiments were done in triplicate. Infections were allowed for 24 hours after which the medium was replaced with fresh U2OS medium. After a further 24 hours, cells were washed with Phosphate Buffered Saline (PBS) and frozen at -20°C in 100 µl of PBS.
After thawing and resuspension of the cell lysate, 75 µl of each well was transferred to a fresh plate, 75 µl of Steady-Glo (Promega) was added and incubated for 15 minutes at room temperature. 100 µl of the resulting mixture was transferred to Wallac Black&White sample plates and luciferase activity was determined on a Wallac Trilux 1450 microbeta Liquid Scintillation and Luminescence Counter.

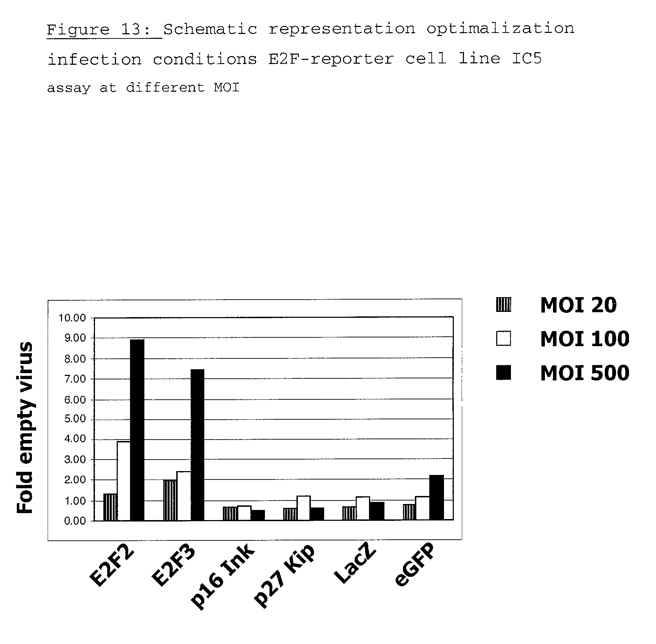

Results are summarized in Figure 13. Obviously, an MOI of 500 for E2F2 and E2F3 gives the highest induction of the E2F-luciferase reporter. Repression by p16Ink and p27Kip is more difficult to monitor, but the highest repression is also seen with the highest MOI.

In a further experiment, we analysed whether the length of incubation after infection would influence the outcome of the experiments.

5x10³ U2OS-1C5 cells were seeded per well in a 96 well plate, using U2OS medium, and incubated overnight at 37°C in a humidified incubator at 10% CO2. A total of two plates were used.

After 24 hours, cells were infected with adenoviruses transducing E2F2, E2F3, p16INK, p27Kip, LacZ, eGFP and empty. MOI used were 100 and 500. All experiments were done in triplicate on the two plates. Infections were allowed for 24 hours after which the medium was replaced with fresh U2OS medium. After a further 24 hours, one of the plates was washed with PBS and frozen at -20°C in 100 µl of PBS. The remaining plate was washed and frozen 24 hours later.
After thawing and resuspension of the cell lysate, 75 µl of each well was transferred to a fresh plate, 75 µl of Steady-Glo (Promega) was added and incubated for 15 minutes at room temperature. 100 µl of the resulting mixture was transferred to Wallac Black&White sample plates and luciferase activity was determined on a Wallac Trilux 1450 microbeta Liquid Scintillation and Luminescence Counter.

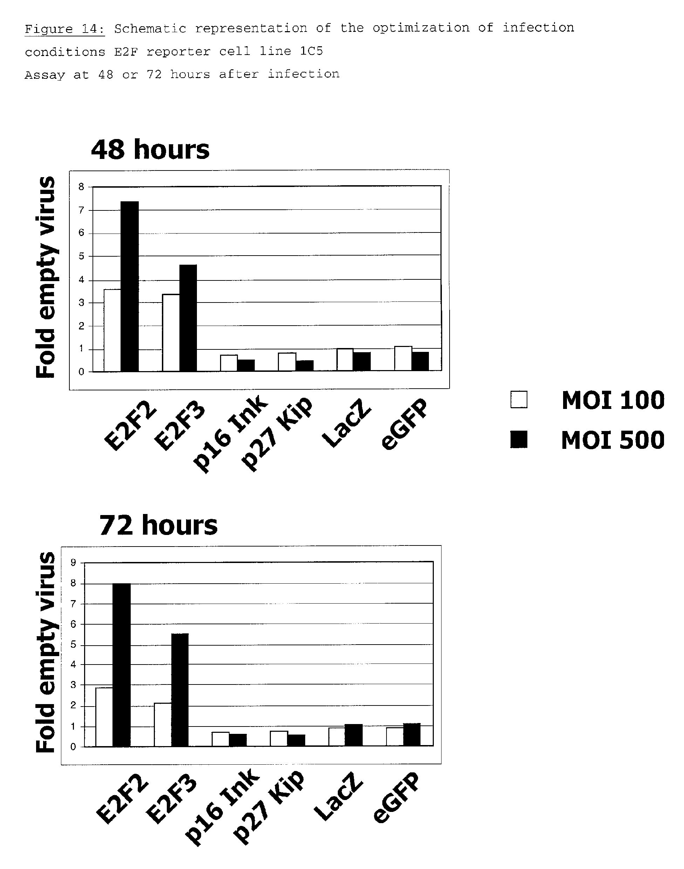

Results are summarized in Figure 14. As can be seen in these figures, activation of the E2F-reporter by E2F2 and E2F3 is comparable between 48 hours and 72 hours infection time. However, repression by p16INK and p27Kip is more pronounced after 48 hours compared to 72 hours. It therefore was concluded that the optimal length of infection is 48 hours.

In an attempt to make repression of the E2F-luciferase reporter by p16INK and p27Kip more pronounced, we performed co-infection experiments with different MOI of E2F2 to enhance the basic expression of the reporter. In the same experiment, the effect of reducing the amount of FBS from 10% to 2% was examined.

For this, 5x10³ U2OS-1C5 cells were seeded per well in a 96 well plate, using U2OS medium, and incubated overnight at 37°C in a humidified incubator at 10% CO2. A total of 3 plates were seeded.

The next day, plate 1 was infected with adenoviruses transducing E2F3, p16INK, p27Kip, LacZ, eGFP, empty, and pClip-L61Ras. MOI used were 100 and 500, each in triplicate, using half of the plate. Infections were duplicated on the second half of the plate. The same layout was used to infect plate two and three. However, all wells from plate 2 were co-infected with MOI 20 of adenovirus transducing E2F2, while all wells of plate 3 were co-infected with MOI 100 of adenovirus transducing E2F2.

Infections were allowed for 24 hours after the medium on the first half of the plates was replaced with fresh U2OS medium, while on the second half of the plates, it was replaced with U2OS-medium containing 2% FBS. After a further 24 hours, all plates was washed with PBS and frozen at -20°C in 100 µl of PBS.

After thawing and resuspension of the cell lysate, 75 µl of each well was transferred to a fresh plate, 75 µl of Steady-Glo (Promega) was added and incubated for 15 minutes at room temperature. 100 µl of the resulting mixture was transferred to Wallac Black&White sample plates and luciferase activity was determined on a Wallac Trilux 1450 microbeta Liquid Scintillation and Luminescence Counter.

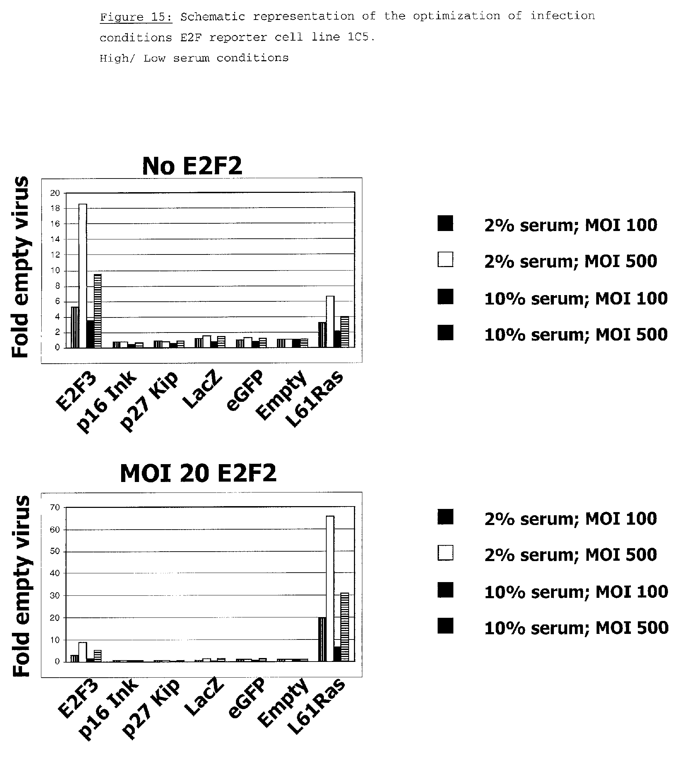

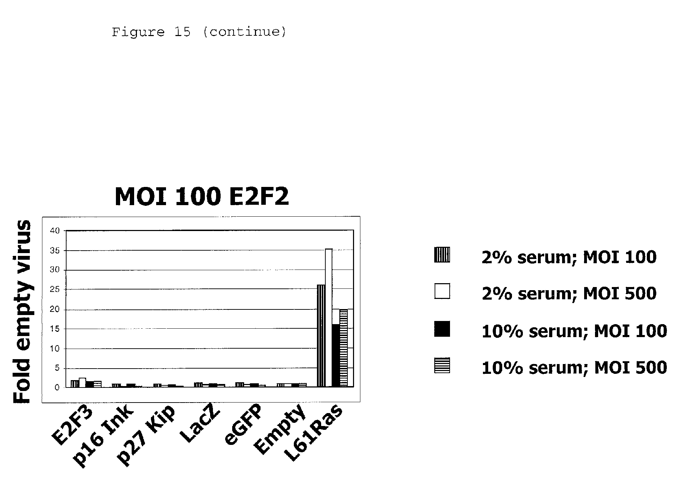

Results are summarized in Figure 15. Induction of the E2F-luciferase reporter by E2F3 and L61Ras is MOI-dependent, with more induction at higher MOI, and is more pronounced at 2% FBS of than at 10% FBS. Repression by p16INK and p27Kip does not differ significantly between the two growth conditions.

When co-infected with MOI 20 of E2F2, the basic signal is higher than without co-infection and the fold induction over empty virus is less for E2F3. This effect is even higher when co-infecting with MOI 100 of E2F2.
L61Ras, however, seems to co-operate with E2F2 in that the fold induction over empty virus is dramatically increased when co-infected with MOI 20 or 100 of E2F2. The induction by L61Ras, co-infected with MOI 20 or 100 of E2F2, is even 5 fold higher than the induction by E2F3 after co-infection with MOI 20 or 100 of E2F2, while induction of L61Ras in the absence of E2F2 is less than that of E2F3. This suggests some synergism between the Ras- and E2F-pathways.

Co-infection with E2F2 did not clearly result in a more pronounced repression of the E2F-luciferase by p16INK and p27Kip.

Therefore, since the effects of serum reduction and co-infection of E2F2 did not result in more pronounced reduction of the E2F-luciferase reporter by p16INK and p27Kip, these conditions were not used for the screenings.

### Example 4. E2F screen with 1500 adenoviruses in 96 well format

To determine the feasibility of the E2F-reporter assay, a random 1440 viruses of the placenta library were picked and used to infect the U2OS 1C5 reporter cell line.

For this, U2OS 1C5 reporter cells were seeded at a density of 5x10³ cells per well in a 96 well plate and incubated overnight at 37°C in a humidified incubator at 10% CO2 in 100 µl of DMEM supplemented with 10% heat inactivated FBS.
The next day, cells were infected with 10 µl of crude lysate of 15 cherry picked propagated virus plates of the adenoviral placenta library in a total volume of 20 µl. The assumed titre of this library is 5x10⁸ virus particles per ml, resulting in a MOI of 1000.
Control viruses, transducing p16INK, p27Kip, E2F2, E2F3, eGFP, and Empty, were included at known MOI of 10, 100, and 1000 in duplo. Two independent virus preparations were used for p16INK, p27Kip, E2F2, and E2F3.

24 hours after infection, the medium of the 96 well plates was replaced with 100 µl of fresh medium.

48 hours after infection, the medium was removed from the wells and the cells were washed once with Phosphate Buffered Saline and frozen at -20°C in 100 µl of PBS.

After thawing and resuspension of the cell lysate, 50 µl of each well was transferred to a Wallac Black&White sample plate and 50 µl of Steady-Glo (Promega) was added and incubated for 15 minutes at room temperature. Luciferase activity was determined on a Wallac Trilux 1450 microbeta Liquid Scintillation and Luminescence Counter.

The whole experiment was performed twice. Empty virus gave mean luciferase readings of 17.3 and 15.6 relative light units, respectively, in the two experiments, with standard deviations of 2.6, and 2.2, respectively. At MOI 10, E2F2 and E2F3 expression caused a 1.5 to 3.1 increase of the luciferase signal, compared to empty virus control. At MOI 100, E2F2 and E2F3 expression caused a 2.3 to 8.3 fold induction of the luciferase signal, compared to empty virus control. At MOI 1000, induction by E2F2 and E2F3 was between 7.1 and 10.9 fold empty virus.
Repression by p16INK and p27Kip was more difficult to monitor. In general, the highest MOI resulted in the highest repression. At MOI 1000, the mean repression by p16INK was 0.7 fold empty virus, while p27Kip expression resulted in a 0.5 fold decrease of the signal of empty virus.

The mean signal of the library was 17.3 and 15.9, respectively, for the two experiments, with standard deviations of 10.5 and 21.6, respectively.

Individual wells were selected that gave in both experiments luciferase readings higher than the mean of empty virus plus 4 times the standard deviation, which values are 27.6 and 24.5, respectively. A total of 18 potential activators were selected.

Individual wells were also selected that gave in both experiments luciferase readings lower than the mean of empty virus minus 4 times the standard deviation, which values are 7.0 and 6.7, respectively. A total of 3 potential repressors were selected.

All 21 potential hits were subjected to a second round of screening (see example 5).

### Example 5. Rescreen of hits from 1500 screen

To propagate the viruses used in the E2F assay, 2.25 x 10⁴ Per.C6/E2A cells were seeded in 200 µl of DMEM containing 10% non-heat inactivated FBS into each well of a 96 well plate and incubated overnight at 39°C in a humidified incubator at 10% CO2. Subsequently, 10 µl of crude lysate, containing the viruses from the placenta library, was added and incubation was proceeded at 34°C in a humidified incubator at 10% CO2 for 8 days, after which the plates were frozen at -20°C.

To rescreen the 21 potential hits from the first round, U2OS 1C5 reporter cells were seeded in 96 well plates at a density of 5x10³ cells per well using 100 µl of DMEM supplemented with 10% heat inactivated FBS.

The next day, cells were infected in triplicate using an MOI of 100 and 500 and a total infection volume of 20 µl.

Infections were done with the 21 potential hits as identified in the first round of screening (see example 4) and 21 randomly picked viruses from the same plates as control. We assumed a titer of 5x10⁹ virus particles (vp) per ml for the propagated viruses from the library. Known titers were used for the control viruses transducing E2F2, E2F3, pl6INK, p27Kip, LacZ, eGFP and empty. Viruses transducing E2F2, p16INK and empty, were included on all 96 well plates.

24 hours after infection, the medium of the 96 well plates was replaced with 100 µl of fresh medium.

48 hours after infection, the medium was removed from the wells and the cells were washed once with Phosphate Buffered Saline supplemented with 1 mM Ca²⁺ and 1 mM Mg²⁺ (PBS⁺⁺), and frozen away at -20°C in 100 µl of PBS⁺⁺.

After thawing and resuspension of the cell lysate, 75 µl of each well was transferred to a fresh plate, 75 µl of Steady-Glo (Promega) was added and incubated for 15 minutes at room temperature. 100 µl of the resulting mixture was transferred to a Wallac Black&White sample plate and luciferase activity was determined on a Wallac Trilux 1450 microbeta Liquid Scintillation and Luminescence Counter.

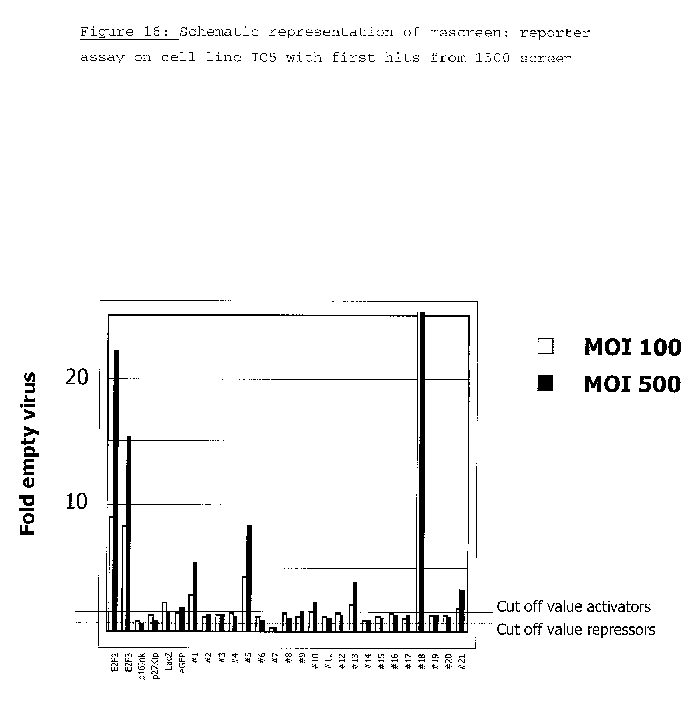

Results were calculated as fold activation compared to empty virus. Of the 21 potential hits tested (see Figure 16), 6 were retained that stimulated E2F-reporter activity:#1 (198227 G02); #5 (198237 B06); #10 (198242 C10); #13 (198247 D10); #18 (198277 C04); and #21 (198282 G05)]. The level of activation differed considerable between the potential hits, with #18 being the strongest inducer. One potential hit was retained that repressed E2F-reporter activity [#7 (198237 F11)]. All controls used in this assay gave good results in that E2F2 and E2F3 stimulated the luciferase reporter 8-20 times compared to empty virus in a MOI-dependent manner; p16INK and p27Kip repressed luciferase activity 0.8-0.6 times compared to empty virus in a MOI-dependent manner; while other control viruses like LacZ and eGFP hardly influenced luciferase activity. The randomly picked viruses from the propagated plates likewise hardly influenced luciferase activity (data not shown).

### Example 6. Validation hits from rescreen 1500

To analyse whether the activation or repression of the luciferase signal after infection of the 7 potential hits in the E2F-reporter cell line U2OS 1C5 (see example 5), was mediated through the E2F-binding sites in the promoter of the reporter, and not through plasmid or genomic sequences flanking the integrated reporter construct, the E2F-luciferase reporter construct and a control reporter construct were transiently transfected in wildtype U2OS cells.

Potential hits that were retained after the rescreen include #1 (198227 G02); #5 (198237 B06); #10 (198242 C10); #13 (198247 D10); #18 (198277 C04); #21 (198282 G05); and #7 (198237 F11). Particle titers of these viruses were determined by real-time PCR, as described (Ma et al., 2001).

For the transient reporter assay, 3 x 10⁵ U2OS cells were seeded in each well of a 6 well plate in 2 ml of DMEM + 10% heat inactivated Foetal Bovine Serum (U2OS-medium).

The next day, medium was replaced with 1.65 ml of fresh U2OS medium. 2 hours later, individual wells of the 6 well plate were transfected with either the E2F-luciferase reporter construct, or the pGL3-basic control reporter construct. Transfection was performed using the Calcium Phosphate Transfection System according to the manufacturer's protocol (Life Technologies). However, all volumes were adjusted (divided by 6.05), since the protocol is described for a 100 mm tissue culture dish instead of a 6 well dish. The total amount of DNA was 3.3 microgram per well, and identical amounts of reporter DNA and carrier DNA were used. The precipitate was left for 24 hours on the wells.

After 24 hours, cells harvested with Trypsine/EDTA (Life Technologies) and collected in U2OS medium according to standard procedures. 5 x 10³ transfected U2OS cells were seeded per well in a 96 well plate in 100 µl of U2OS-medium and incubated overnight at 37°C in a humidified incubator at 10% CO2.

The next day, viruses encoding potential hits (see above) and control viruses transducing E2F2, p16INK, p27Kip, eGFP, *Lac*Z, and Empty, were used to infect U2OS cells transiently transfected with the E2F-luciferase reporter construct, or the pGL3-basic control reporter construct. Cells were infected with the viruses at MOI of 100 and 500. 6 wells of a 96 well plate were used for each MOI for all viruses. Cells were incubated further for 48 hours at 37°C in a humidified incubator at 10% CO2.

48 hours after infection, the medium was removed from the wells and the cells were washed once with PBS and frozen away at -20°C in 100 µl of PBS.

After thawing and resuspension of the cell lysate, 50 µl of each well was transferred to a Wallac Black&White sample plate and 50 µl of Steady-Glo (Promega) was added and incubated for 15 minutes at room temperature. Luciferase activity was determined on a Wallac Trilux 1450 microbeta Liquid Scintillation and Luminescence Counter.

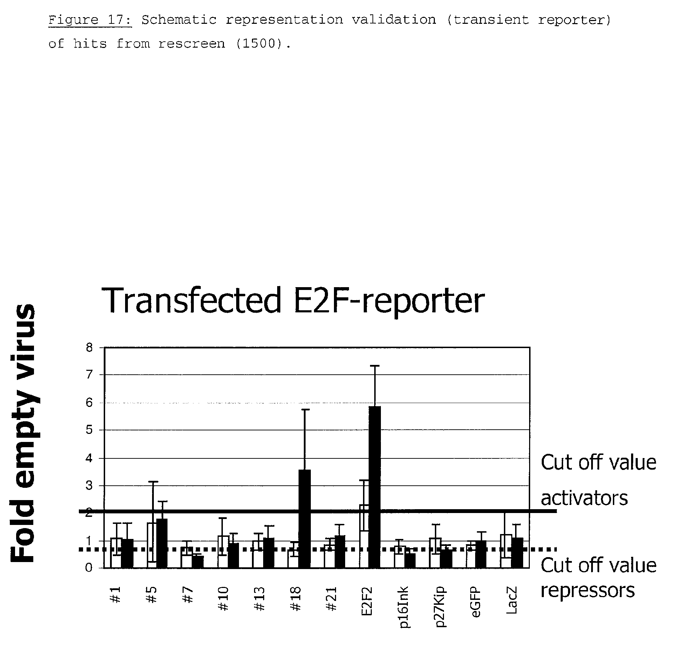

Results are presented relative to empty virus control in Figure 17. Neither of the potential hits, nor the control viruses, modulated expression of the transfected pGL3-basic control reporter construct (data not shown).
Activation of the transfected E2F-luciferase reporter construct (Figure 17) was achieved by E2F2 (MOI dependent; maximal induction of 5.8 times empty vector) and hit #18 (MOI dependent; maximal induction of 3.5 times empty vector). All other potential hits scored below two times empty vector at both MOI and were not considered further.

Repression was mediated by transduction of p16INK and p27Kip (MOI dependent; maximal repression of 0.55 and 0.64 times empty vector, respectively) and hit #7 (MOI dependent; maximal repression of 0.44 times empty vector).

Thus it was concluded that 2 hits (#7, a repressor; #18, an activator) could be validated on a transiently transfected E2F-reporter construct. None of the 2 hits modulated expression of a transiently transfected control reporter plasmid (data not shown).

### Example 7. E2F screen with 11.000 viruses in 384 well format

### Preparation of the control plates

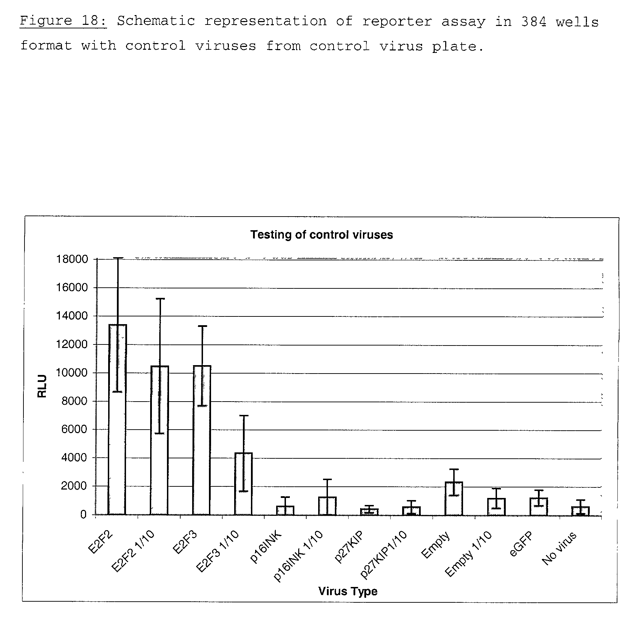

Control plates were prepared that contained different control pIPspAdApt viruses transducing the following transgenes: E2F2, E2F3, p16INK, p27KIP, GFP or the empty virus (defined as the virus with empty MCS) or no virus at all. These viruses were propagated according to the protocol applied for the Phenoselect library. Day 0, TC treated 96 well plates were seeded with PerC6/E2A cells at a density of 2,25x10⁴ cells per well in 200 µl medium. Day 1, 48 wells per plate were infected with 20 µl of one type of control virus emanating from a larger batch preparation. After 7 days, full CPE was obtained. The plates were subjected to one freeze-thaw cycle and the crude virus lysate was aliquoted in 96 well V-bottom plates as follows. The 8 wells of every column were filled with 25 µl of one type of control virus (See Figure 18).

Column 1: E2F2 virus. Column 2: 1/10 dilution of the E2F2 virus. Column 3: E2F3 virus. Column 4: 1/10 dilution of the E2F3 virus. Column 5: p16INK.

Column 6: 1/10 dilution of the p16INK virus. Column 7: p27KIP virus. Column 8: 1/10 dilution of the p27KIP virus. Column 9: Empty virus. Column 10: 1/10 dilution of the empty virus. Column 11: GFP virus. Column 12: Medium + 10% FBS.

The aliquots were sealed with a seal (Nunc Cat N° 236366) and stored at -80°C until use.

The control plates were tested according to the screening protocol. 8 µl of virus crude lysate was pipetted from a control plate using a 96 channel Hydra dispensor (Robbins Scientific) and 1 µl was dispensed in positions A1, A2, B1 and B2 of a white 384 well plate (Greiner) in which U2OS 1C5 reporter cells were seeded at a density of 1250 cells/well (20 µl medium per well) . 48 hrs post-infection, 15 µl of Luciferase substrate (Promega Steady Glow) was added to the wells, the plates were sealed and put on a rotary shaker for 30 min. Readout was then performed in a luminometer (Lumicount, Packard, Gain 150, PMT voltage 1100V). Results are shown in Figure 18. For the undiluted virus controls, E2F expression causes a 5.8-fold (E2F2) or 4.5-fold (E2F3) rise of the signal as compared to the empty virus infected wells. A 4-fold or 5-fold reduction of the signal was seen when expressing p16INK or p27KIP, respectively. A 10-fold dilution of the control viruses resulted in a 8.8-fold and 3.7 fold activation of the signal as compared to the wells infected with the empty virus for the E2F2 and E2F3 viruses, respectively and zero or a 2-fold reduction of the signal as compared to the empty virus infected wells for p16INK and p27KIP respectively. This experiment confirmed the quality of the produced control plates and yielded the trends observed previously.

### Protocol for screening of the PhenoSelect library

U2OS reporter cells 1C5 were cultured in DMEM containing 10% of heath inactivated FBS and 1 µg/ml puromycin. Performing the assay, U2OS cell cultures were strictly kept subconfluent.
Day -3, 5 T175 flasks were seeded with U2OS reporter cells C15 at a density of 1.5x10⁶ cells per flask.
Day 0, T175 flasks seeded day -3 were treated with trypsin/EDTA (2ml of trypsin/EDTA mix/flask) to detach cells. Cells (resuspended in 10 ml culture medium/T175 culture flask) were counted. Cells were then resuspended in culture medium at a density of 6,25x10⁴ cells/ml for further seeding. White tissue culture treated 384 well plates were seeded at a density of 1,25x10⁴ cells per well, 20 µl per well, using a multidrop (Labsystems).
Day 1, approximately 18 hours after seeding of the reporter cells, reporter cells were infected with the library viruses as follows.

The virus library aliquot plates (384 well format) planned to be processed (10 plates per day) were put in a laminar air flow cabinet for 1 hour for thawing. Plates are then put at 4°C until further processing.

For every well of the 384 virus library aliquot plate, 1 µl of virus crude lysate was transferred to three wells (coordinates A1, A2 and B1) of the white 384 well plate containing the seeded reporter cells. This was done using a Hydra 96 dispenser (110 µl) (Robbins Scientific). The pipettor is programmed to fill its syringes with 10 µl of virus crude lysate and to dispense 1 µl at positions A1, A2 and B1 in the plate containing the reporter cells. After this action, syringes were emptied in the original virus library aliquot plate. Before processing of the following virus library aliquot plate, syringes were cleaned by performing 3 washing steps with 20 µl of 5% bleach. The syringes are then rinsed 3 times with 25 µl of sterile deionized water.

After processing of all virus library aliquot plates, the control viruses were added to the plates as follows: for every well of the 96 well control plate, one µl of virus crude lysate was transferred to 1 well, B2 quadrant, on 8 to 10 384 well plates containing the reporter cells infected with the library viruses. (This position was left uninfected during infection of the reporter cells with the library viruses.) Addition of the control viruses was also performed using the Hydra dispenser.

Approximately 48 hours after infection, readout of reporter activation was performed. The luciferase substrate (Steady Glow, Promega) was freshly prepared according to the protocol of the manufacturer. 15 µl of luciferase substrate was added to the wells using the Hydra dispenser. This operation was performed in a laminar air flow cabinet and under subdued light conditions. The dispenser was programmed to fill its syringes with 70 µl of substrate and to sequentially dispense 15 µl to the A1, A2, B1 and B2 quadrants. The syringes were then refilled for processing the next plate without intermediate washing step. After addition of the substrate, the plates were sealed (Nunc cat N° 236366) and put on a rotary shaker for 30 min. Plates were then sequentially inserted into a luminometer (Lumicount, Packard) for readout. The apparatus was used with the following settings: Gain 150, PMT voltage 1100 V, 0.3 sec reading time. Time in between substrate addition and readout was not allowed to exceed 1 hour. Data were stored in Excel sheet format (Microsoft).

The screening was performed in 4 series of 10x384 well virus aliquot plates, which represented 15360 wells. As the virus production efficiency for the Phenoselect library reached on average 70% of the total amount of wells, this represented approximately 10750 viruses.

### Data analysis.

The data obtained from the luminometer were analysed as follows.

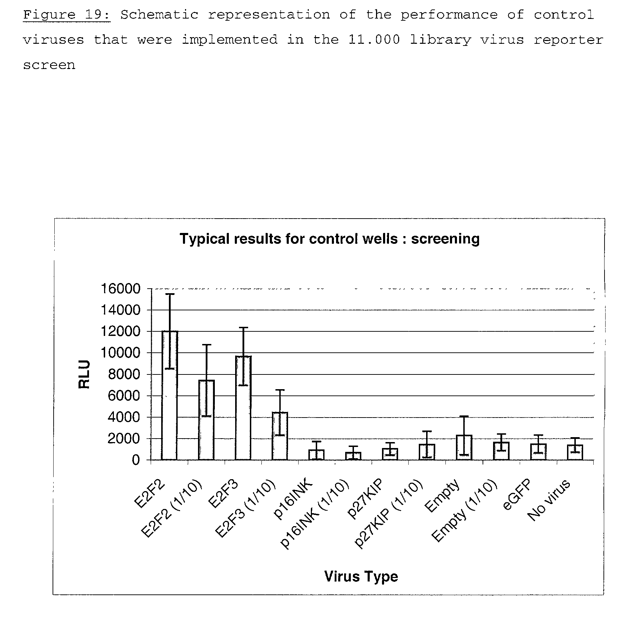

In first instance, the control data inserted in 96 positions of the B2 quadrant in 8 to 10 assay plates per screen were extracted and compiled. Background signal levels associated with the Empty virus and the standard deviation on this measurement were determined. The results obtained for the wells infected with the various control viruses were analysed in order to evaluate the quality of the screening. A typical result for the wells infected with the control viruses during one out of the 4 runs of the screening is shown in Figure 19. As 8 wells of the control plate contained the same virus, and as reporter cells in 8 to 10 screening plates were infected with the control viruses, each control virus was tested at least 64 times per run. The mean of the 3 values obtained for every individual library virus was calculated. All mean values were sorted. Viruses causing an increase of the signal are considered as hits provided these mediated a signal superior to the cut off value. The cut off value for samples identified as 'E2F activators' was defined as being the mean plus three times the standard deviation of the signal obtained for the wells in which cells were infected with the empty virus. Library viruses that mediated a lower signal as the empty virus-infected wells were considered as hits provided these mediated a signal of at least half of the signal of the 8 neighbour library viruses.

### Example 8. Rescreen of hits from 11.000 screen

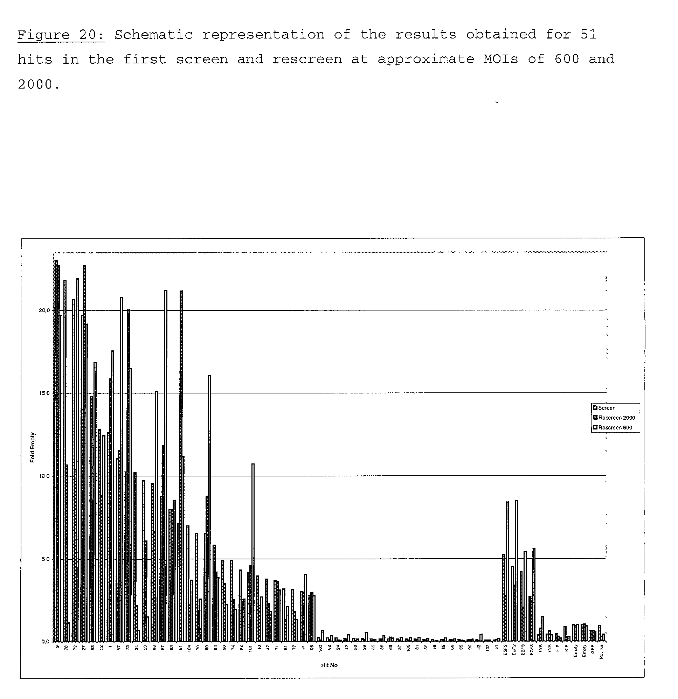

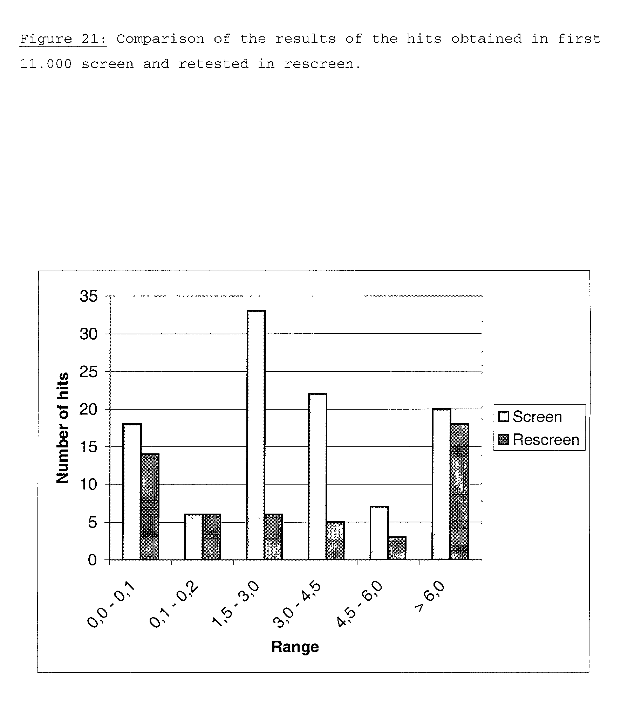

For the viruses scored as hit, two µl of virus crude lysate was recovered from the well of the original 384 well aliquot plates that were used for performing the screening. These aliquot plates were stored at -80°C and thawed for a second time for removal of this 2 µl aliquot. The viruses of the hits were propagated by using the 2 µl aliquots of crude virus to infect 2,25x10⁴ Per.C6/E2A cells seeded in 96 well plates (200µl of DMEM + 10% FBS). After appearance of complete CPE, these 96 well plates underwent a single freeze-thaw cycle. Four aliquots of 40 µl (stored in V-bottom 96 well plates) were prepared from the 200 µl of supernatant of the infected Per.C6/E2A cells. These aliquots were used for performing the rescreen. The aim of the rescreen was to test the repropagated 'hit viruses' using the stable reporter cell line 1C5 at various MOIs. This rescreen was performed applying the same protocol as the one used for the primary screen (see example 7). Briefly, 1 µl of the undiluted virus crude lysate aliquots (emanating from the repropagation step) as well as 1 µl from a 3-fold dilution of these aliquots were used to infect the 1C5 U2OS reporter cell line seeded in 384 well plates. (This corresponds to MOI of approximately 2000 and 600, respectively). Two days after infection, luciferase substrate was added and readout was performed. Results of the rescreen were compared to the results of the original screening (Figure 20; Remark: for clarity of the graph, the value indicated for hit 9 at MOI 600 corresponds to one fourth of the real value and the value indicated for hit 27 at MOI 600 corresponds to one third of the real value.) The cut off value for samples identified as `E2F activators' was defined as being the mean plus three times the standard deviation of the signal obtained for the wells in which cells were infected with the empty virus. The viruses mediating a signal lower as the non-infected wells (indicated as 'No virus') were scored as repressors. Applying these cut off values, 27 of the hits were confirmed as activators and 21 hits were confirmed as repressors for the higher MOI. At the lower MOI, 22 hits were confirmed as activators and 15 as repressors. The distribution of the 106 hits obtained in the original screening is represented in Figure 21. Two ranges were defined for the repressors (One fifth to one tenth or less as one tenth of the empty virus signal) and 4 ranges for the activators (1.5 to 3 fold, 3 to 4.5 fold, 4.5 to 6 fold or more as 6 fold the empty virus signal). In the same graph, the number of hits within the different ranges that were confirmed during the rescreen (at the approximate MOI of 2000) were indicated. From these data, we can conclude that most repressors could be confirmed in the rescreen. For what concerns the activators, the strongest hits (more as 6 fold activation) were generally confirmed, the moderate activators (between 4.5 and 6 fold empty virus) were confirmed in 50 % of the cases and the weak activators (less as 4.5 fold empty virus) were generally not confirmed.

### Example 9. Validation hits from rescreen 11000

To propagate the potential hits of the E2F assay, 2.25 x 10⁴ Per.C6/E2A cells were seeded in 200 µl of DMEM containing 10% non-heat inactivated FCS into each well of a 96 well plate and incubated overnight at 39°C in a humidified incubator at 10% CO2. Subsequently, 5 µl of crude lysate, containing the viruses from the placenta library, was added to two of the wells and incubation was proceeded at 34°C in a humidified incubator at 10% CO2 for 12 days, after which the plates were frozen at -20°C.

Particle titers of these viruses were determined by real-time PCR, as described (Ma et al., 2001).

For the transient reporter assay, 3 x 10⁵ U2OS cells were seeded in each well of a 6 well plate in 2 millilitre of DMEM + 10% heat inactivated Foetal Calf Serum (U20S-medium).

The next day, medium was replaced with 1.65 ml of fresh U2OS medium. 2 hours later, individual wells of the 6 well plate were transfected with either the E2F-luciferase reporter construct, or the pGL3-basic control reporter construct (Promega), or the pGL3-promoter control reporter construct (Promega). Transfection was performed using the Calcium Phosphate Transfection System according to the manufacturer's protocol (Life Technologies). However, all volumes were adjusted (divided by 6.05), since the protocol is described for a 100 mm tissue culture dish instead of a 6 well dish. The total amount of DNA was 3.3 microgram per well, and identical amounts of reporter DNA and carrier DNA were used. The precipitate was left for 24 hours on the wells. After 24 hours, cells harvested with Trypsine/EDTA (Life Technologies) and collected in U2OS medium according to standard procedures. 5 x 10³ transfected U2OS cells were seeded per well in a 96 well plate in 100 µl of U2OS-medium and incubated overnight at 37°C in a humidified incubator at 10% CO2.

The next day, re-propagated viruses encoding potential hits (see above) and control viruses transducing E2F2, p16Ink, p27Kip, eGFP, LacZ, and Empty, were used to infect U2OS cells transiently transfected with the E2F-luciferase reporter construct, or the pGL3-basic or pGL3-promoter control reporter constructs. Cells were infected with the viruses at MOI of 100 and 500. 3 wells of a 96 well plate were used for each MOI for all viruses. Cells were incubated further for 48 hours at 37°C in a humidified incubator at 10% CO2.

48 hours after infection, the medium was pulled of from the wells. The cells were washed once with PBS and frozen away at -20°C in 100 µl of PBS.

After thawing and resuspending of the cell lysate, 50 µl of each well was transferred to a Wallac Black&White sample plate and 50 µl of Steady-Glo (Promega) was added and incubated for 15 minutes at room temperature. Luciferase activity was determined on a Wallac Trilux 1450 microbeta Liquid Scintillation and Luminescence Counter.

Results were calculated as fold activation compared to empty virus.

All controls used in this assay gave good results in that E2F2 and E2F3 stimulated the E2F-luciferase reporter 4.5-9 times compared to empty virus while p16Ink and P27Kip repressed luciferase activity 0.4-0.2 times compared to empty virus in a MOI-dependent manner. Other control viruses like eGFP hardly influenced luciferase activity.

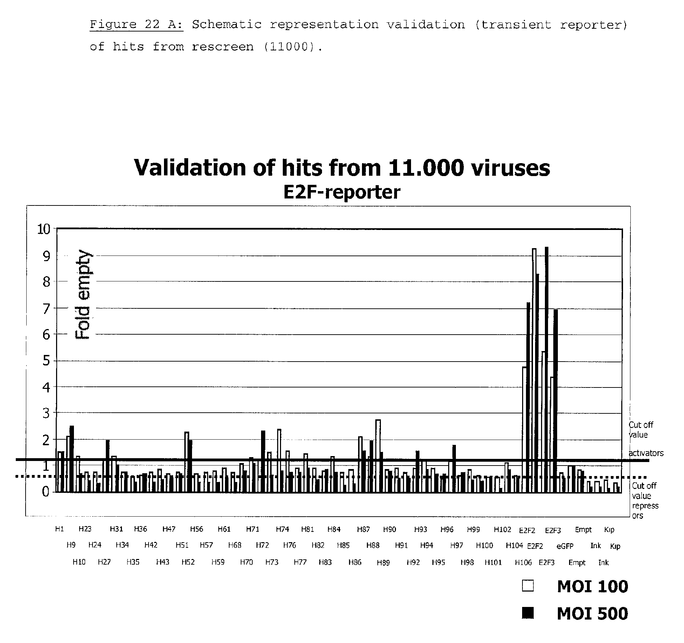

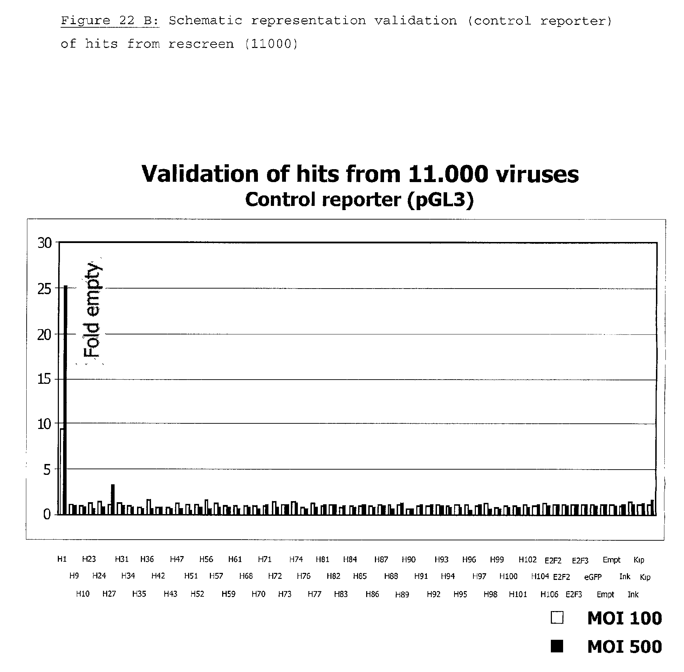

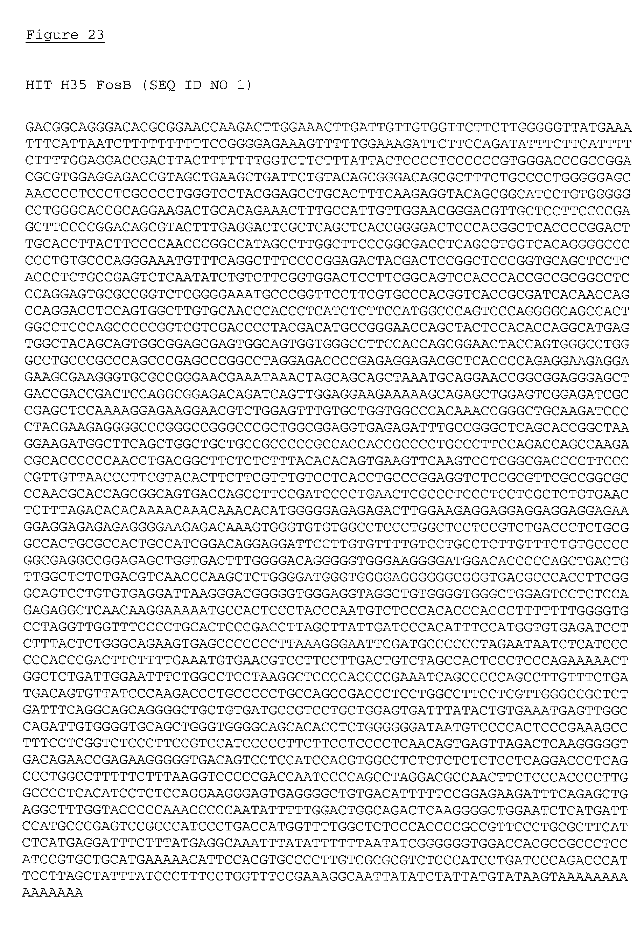

Of the 51 potential hits tested (see Figure 22A), 7 were retained that stimulated E2F-reporter activity more than 1.2 times the value of empty vector (H9; H52; H72; H74; H87; H88; H97), and which did not stimulate the pGL3-basic or pGL3-promoter control reporters (Figure 22B and data not shown).

Two potential hits (H1 and H27) stimulated both the E2F reporter and the pGL3-basic control reporter (compare Figures 22A and 22B), and were discarded. Two potential hits (H89 and H92) stimulated both the E2F reporter and the pGL3-promoter control reporter to equal relative levels and were also discarded

Eleven potential hits were retained that repressed E2F-reporter activity more than 0.6 times empty vector control (H24, H35, H56, H57, H59, H82, H85, H96, H100, and H106), while not influencing the pGL3-basic or pGL3-promoter control reporters (see Figure 22A). Several other potential repressors were discarded since they also seemed to influence the pGL3-promoter control reporter (data not shown).

### Example 10. Sequence identification of validated hits

For sequencing and sample tracking purposes, fragments of the cDNAs expressed by the hit adenoviruses were amplified by PCR using primers complementary to sequences flanking the MCS of the pAdapt plasmid. The following protocol was applied to obtain these PCR fragments. Day 0, PerC6/E2A cells were seeded in 96 well plates at a density of 2,25x10⁴ cells per well, in 200 µl of PerC6/E2A medium. Cells were incubated overnight at 39°C, 10% CO₂. Day 1, cells were infected with the 'hit viruses' using 2 µl of crude cell lysate material from the repropagation step. Cells were then incubated at 34°C, 10% CO₂ until appearance of starting of CPE (as revealed by the swelling and rounding up of the cells, typically 2 to 3 days post infection). The supernatant was then removed from the cells and 50 µl of lysis buffer (1X Expand High Fidelity buffer with MgCl₂ (Roche Molecular Biochemicals Cat N° 1332465) supplemented with 1 mg/ml proteinase K (Roche Molecular Biochemicals Cat N° 745 723) and 0.45% Tween-20 (Roche Molecular Biochemicals, Cat N° 1335465) was added to the cells. Cell lysates were then transferred to sterile micro centrifuge tubes and incubated at 55°C for 2 hrs followed by a 15 min inactivation step at 95°C. 5 µl of the cell lysates was then added to a PCR master mix composed of 5µl 10X Expand High Fidelity buffer +MgCl2, 1µl of dNTP mix (10mM for each dNTP), 1µl of pClip-FOR primer (10µM stock, sequence: 5' GGT GGG AGG TCT ATA TAA GC), 1µl of pAdapt-REV primer (10µM stock, sequence: 5' GGA CAA ACC ACA ACT AGA ATG C), 0.75 µl of Expand High Fidelity DNA polymerase (3.5U/µl, Roche Molecular Biochemicals) and 36,25 µl of H₂O. PCR was performed using a PE Biosystems Gen Amp PCR system 9700 as follows: the PCR mixture (50µl in total) was incubated at 95°C for 5 min; at 95°C for 30 sec; 55°C for 30 sec; 68°C for 4 min, and this was repeated for 35 cycles. A final incubation at 68°C was applied for 7 min. The amplification products were resolved on a 0.8% agarose gel containing 0,5 µg/ml ethidium bromide and their length estimated by comparison with the migration of a standard DNA ladder. For this purpose, 15µl of PCR mixture was mixed with 10µl of 6X gel loading Buffer. The PCR products obtained were also used as template for sequencing using the aforementioned pClip-FOR primer.

### Example 11. Polynucleotides and polypeptides of the invention

The sequence analysis of the in total 20 identified nucleic acid hits revealed both unknown and known polynucleotide sequences (Table 1). The nuclear receptor PPARgamma, which was isolated in the screenings of the present invention, has already been described as a regulator of E2F and therefore provides an internal control for the screening method of the present invention (Altiok et al., 1997; Wakino et al., 2000).

**Table 1:**

| nucleic acid hits | | | |
|---|---|---|---|
| Hit | Modulator | SEQ. Similarity | SEQ ID |
| #7 | repressor | - | |
| H24 | repressor | ? | |
| H35 | repressor | FOS-B (NM_006732) | 1, 2 |
| H43 | repressor | (NM_018131.1) | |
| H56 | repressor | | |
| H57 | repressor | 5'nucleotidase (X55740.1) | 3, 4 |
| H59 | repressor | (AF226614) | |
| H82 | repressor | UFD1L (NM_005659) | |
| H85 | repressor | | |
| H96 | repressor | PPARgamma (U10374) | |
| H100 | repressor | | |
| H106 | repressor | AF226614.1 | |
| #18 | activator | - | |
| H9 | activator | Hyp.protein (NM_017710) | |
| H52 | activator | | |
| H72 | activator | FEN1 (NM_004111) | |
| H74 | activator | AF087017 | |
| H87 | activator | M25171.1 | |
| H88 | activator | - | |
| H97 | activator | | |

### Features of hit H35

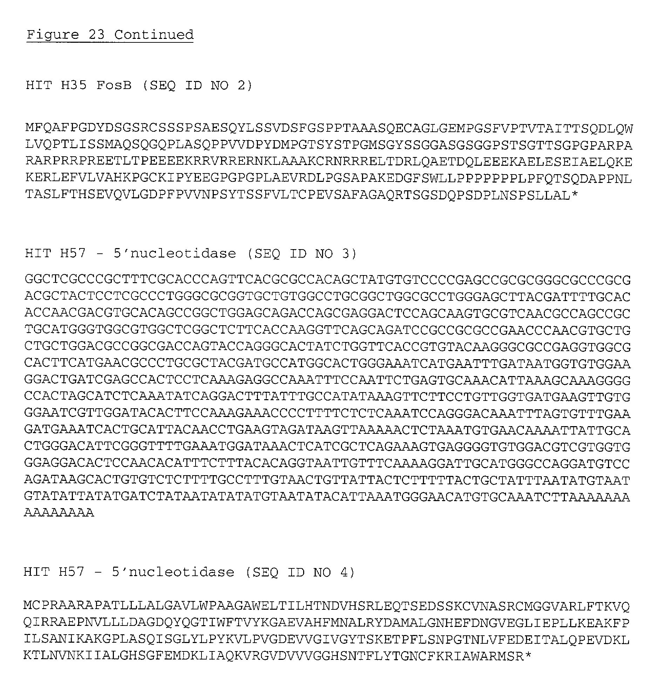

The DNA sequence of hit H35 (see Figure 23; SEQ ID NO 1)is highly similar to a *Homo sapiens* FBJ murine osteosarcoma viral oncogene homolog B (FosB) mRNA, identified by accession number NM_006732. At the DNA level, only three mismatches are observed between the cDNA in H35 (3182 nt) and NM_006732 (3775 nt). A fragment of about 600 nt at the 3' end of NM_006732 is not observed in H35. However, this region is part of the 3' untranslated region of NM_006732. The translation product derived from the DNA sequence of hit H35 (Figure 23; SEQ ID NO 2) is nearly identical to the published coding sequence of FosB: only the C-terminal Arg residue is replaced by a Leu residue in H35. Therefore, it is likely that the adenoviral vector of hit H35 directs the expression of a full-length FosB protein. However, it has been described that a truncated form of FosB, FosB2 can be derived from the full length coding sequence of FosB (US patent 6,008,323). Whether hit H35 results in the translation of this shorter version of FosB needs to be determined.

Fos protein family members (c-Fos, FosB, Fra-1, Fra-2) associate with Jun proteins (c-Jun, JunB and JunD), thereby forming so-called AP-1 transcription factor complexes. Upon growth factor stimulation of quiescent cells, the expression and activity of AP-1 transcription factors is induced. It has been suggested that the cellular targets of the AP-1 transciption factors that regulate cell cycle entry, are the D-type cyclins, which are known regulators of the Rb/E2F pathway (Brown et al., 1998). Thus, the prior art describes Fos proteins as positive regulators of cell proliferation presumably via the activation of Rb/E2F pathway. Based on this, the finding that FosB could be identified as a negative regulator of E2F activity according to the method of the present invention, is new and unexpected.

### Features of hit H57

The DNA sequence of hit H57 (Figure 23; SEQ ID NO 3) is highly similar to human placental cDNA coding for 5'nucleotidase (Genebank accession number X55740.1). The fact that the coding sequences are different in length may be due to alternative transcripts. The N-terminal part of 252/264 amino acid residues of the encoded translation product of H57 (Figure 23; SEQ ID NO 4) is identical to the N-terminus of the 5'nucleotidase protein (574 aa). The C-terminal 12 amino acids of H57 do not match with 5'nucleotidase, probably as a result of alternative splicing. Since hit H57 lacks a large C-terminal portion, compared to the published 5'nucleotidase sequence, in which probably one or more active domains are contained, the activity of hit H57 may differ from the described activities of 5'nucleotidase.

In general, 5'nucleotidase has been considered as a marker enzyme for the plasma membrane, and has been described as a key enzyme in the generation of adenosine by hydrolyzing ATP, thereby playing a role in neurotransmission. However, from its wide range of localization in tissues it is also considered to be related to the membrane movement of cells in the transitional epithelium, cellular motile response, transport process, cellular growth, synthesis of fibrous protein and calcification, lymphocyte activation, and oxygen sensing mechanism (Moriwaki et al., 1999). Furthermore, 5'nucleotidase has been reported as a differentiation marker in differentiation studies using both colon adenocarcinoma and neuroblastoma cells (Kohring and Zimmermann, 1998; Navarro et al., 1997). As such, 5'nucleotidase may play a role in differentiation processes, although the mechanism remains obscure. So far, no direct links between 5'nucleotidase activity and cell cycle regulation have been described. Therefore, the finding, as disclosed in the present invention, that a partial clone of 5'nucleotidase negatively regulates E2F activity, provides new and unexpected insights in 5'nucleotidase function.

### References

Altiok, S., Xu, M. and Spiegelman, B.M. (1997) PPARgamma induces cell cycle withdrawal: inhibition of E2F/DP DNA- binding activity via down-regulation of PP2A. *Genes Dev,* **11,** 1987-98.

Altschul, S.F., Madden, T.L., Schaffer, A.A., Zhang, J., Zhang, Z., Miller, W. and Lipman, D.J. (1997) Gapped BLAST and PSI-BLAST: a new generation of protein database search programs. *Nucleic Acids Res,* **25,** 3389-402.

Beijersbergen, R.L. and Bernards, R. (1996) Cell cycle regulation by the retinoblastoma family of growth inhibitory proteins. *Biochim Biophys Acta,* **1287,** 103-20.

Beijersbergen, R.L., Carlee, L., Kerkhoven, R.M. and Bernards, R. (1995) Regulation of the retinoblastoma protein-related p107 by G1 cyclin complexes. *Genes Dev,* **9,** 1340-53.

Brown, J.R., Nigh, E., Lee, R.J., Ye, H., Thompson, M.A., Saudou, F., Pestell, R.G. and Greenberg, M.E. (1998) Fos family members induce cell cycle entry by activating cyclin D1. *Mol Cell Biol,* **18,** 5609-19.

Claudio, P.P., Fratta, L., Farina, F., Howard, C.M., Stassi, G., Numata, S., Pacilio, C., Davis, A., Lavitrano, M., Volpe, M., Wilson, J.M., Trimarco, B., Giordano, A. and Condorelli, G. (1999) Adenoviral RB2/p130 gene transfer inhibits smooth muscle cell proliferation and prevents restenosis after angioplasty. *Circ Res,* **85,** 1032-9.

Demers, G.W., Harris, M.P., Wen, S.F., Engler, H., Nielsen, L.L. and Maneval, D.C. (1998) A recombinant adenoviral vector expressing full-length human retinoblastoma susceptibility gene inhibits human tumor cell growth. *Cancer Gene Ther,* **5,** 207-14.

Dyson, N. (1998) The regulation of E2F by pRB-family proteins. *Genes Dev,* **12,** 2245-62.

Dzau, V.J., Mann, M.J., Morishita, R. and Kaneda, Y. (1996) Fusigenic viral liposome for gene therapy in cardiovascular diseases [comment]. *Proc Natl Acad Sci U S A,* **93,** 11421-5.

Fagan, R., Flint, K.J. and Jones, N. (1994) Phosphorylation of E2F-1 modulates its interaction with the retinoblastoma gene product and the adenoviral E4 19 kDa protein. *Cell,* **78,** 799-811.

Hagemeier, C., Cook, A. and Kouzarides, T. (1993) The retinoblastoma protein binds E2F residues required for activation in vivo and TBP binding in vitro. *Nucleic Acids Res,* **21,** 4998-5004.

Hannon, G.J. and Beach, D. (1994) p15INK4B is a potential effector of TGF-beta-induced cell cycle arrest [see comments]. *Nature,* **371,** 257-61.

Harper, J.W., Adami, G.R., Wei, N., Keyomarsi, K. and Elledge, S.J. (1993) The p21 Cdk-interacting protein Cip1 is a potent inhibitor of G1 cyclin- dependent kinases. *Cell,* **75,** 805-16.

Hateboer, G., Kerkhoven, R.M., Shvarts, A., Bernards, R. and Beijersbergen, R.L. (1996) Degradation of E2F by the ubiquitin-proteasome pathway: regulation by retinoblastoma family proteins and adenovirus transforming proteins. *Genes Dev,* **10,** 2960-70.

Helin, K. (1998) Regulation of cell proliferation by the E2F transcription factors. *Curr Opin Genet Dev,* **8,** 28-35.

Hsiao, K.M., McMahon, S.L. and Farnham, P.J. (1994) Multiple DNA elements are required for the growth regulation of the mouse E2F1 promoter. *Genes Dev,* **8,** 1526-37.

Ishizaki, J., Nevins, J.R. and Sullenger, B.A. (1996) Inhibition of cell proliferation by an RNA ligand that selectively blocks E2F function. *Nat Med,* **2,** 1386-9.

Johnson, D.G., Cress, W.D., Jakoi, L. and Nevins, J.R. (1994) Oncogenic capacity of the E2F1 gene. *Proc Natl Acad Sci U S A,* **91,** 12823-7.

Johnson, D.G., Schwarz, J.K., Cress, W.D. and Nevins, J.R. (1993) Expression of transcription factor E2F1 induces quiescent cells to enter S phase. *Nature,* **365,** 349-52.

Kohring, K. and Zimmermann, H. (1998) Upregulation of ecto-5'-nucleotidase in human neuroblastoma SH-SY5Y cells on differentiation by retinoic acid or phorbolester. *Neurosci Lett,* **258,** 127-30.

Lukas, J., Herzinger, T., Hansen, K., Moroni, M.C., Resnitzky, D., Helin, K., Reed, S.I. and Bartek, J. (1997) Cyclin E-induced S phase without activation of the pRb/E2F pathway. *Genes Dev,* **11,** 1479-92.

Lundberg, A.S. and Weinberg, R.A. (1998) Functional inactivation of the retinoblastoma protein requires sequential modification by at least two distinct cyclin-cdk complexes. *Mol Cell Biol,* **18,** 753-61.

Luo, R.X., Postigo, A.A. and Dean, D.C. (1998) Rb interacts with histone deacetylase to repress transcription. *Cell,* **92,** 463-73.

Ma, L., Bluyssen, H.A.R., De Raeymaeker, M., Laurysens, V., van der Beek, N., Pavliska, H., van Zonneveld, A.J., Tomme, P. and van Es, H.H.G. (2001) Rapid determination of adenoviral vector titers by quantitative real-time PCR. *Journal of Virological Methods,* **in press.**

Mann, M.J., Whittemore, A.D., Donaldson, M.C., Belkin, M., Conte, M.S., Polak, J.F., Orav, E.J., Ehsan, A., Dell'Acqua, G. and Dzau, V.J. (1999) Ex-vivo gene therapy of human vascular bypass grafts with E2F decoy: the PREVENT single-centre, randomised, controlled trial. *Lancet,* **354,** 1493-8.

Martinez-Balbas, M.A., Bauer, U.M., Nielsen, S.J., Brehm, A. and Kouzarides, T. (2000) Regulation of E2F1 activity by acetylation. *Embo J,* **19,** 662-71.

Marzio, G., Wagener, C., Gutierrez, M.I., Cartwright, P., Helin, K. and Giacca, M. (2000) E2F family members are differentially regulated by reversible acetylation. *J Biol Chem,* **275,** 10887-92.

Morgenstern, J.P. and Land, H. (1990) Advanced mammalian gene transfer: high titre retroviral vectors with multiple drug selection markers and a complementary helper-free packaging cell line. *Nucleic Acids Res,* **18,** 3587-96.

Moriwaki, Y., Yamamoto, T. and Higashino, K. (1999) Enzymes involved in purine metabolism--a review of histochemical localization and functional implications. *Histol Histopathol,* **14,** 1321-40.

Morris, L., Allen, K.E. and La Thangue, N.B. (2000) Regulation of E2F transcription by cyclin E-Cdk2 kinase mediated through p300/CBP co-activators. *Nat Cell Biol,* **2,** 232-9.

Muller, H. and Helin, K. (2000) The E2F transcription factors: key regulators of cell proliferation. *Biochim Biophys Acta,* **1470,** M1-12.

Muller, H., Moroni, M.C., Vigo, E., Petersen, B.O., Bartek, J. and Helin, K. (1997) Induction of S-phase entry by E2F transcription factors depends on their nuclear localization. *Mol Cell Biol,* **17,** 5508-20.

Navarro, J.M., Olmo, N., Turnay, J., Lopez-Conejo, M.T. and Lizarbe, M.A. (1997) Differentiation of BCS-TC2 human colon adenocarcinoma cells by sodium butyrate: increase in 5'-nucleotidase activity. *Eur J Clin Invest,* **27,** 620-8.

Peeper, D.S., Keblusek, P., Helin, K., Toebes, M., van der Eb, A.J. and Zantema, A. (1995) Phosphorylation of a specific cdk site in E2F-1 affects its electrophoretic mobility and promotes pRB-binding in vitro. *Oncogene,* **10,** 39-48.

Peeper, D.S., Upton, T.M., Ladha, M.H., Neuman, E., Zalvide, J., Bernards, R., DeCaprio, J.A. and Ewen, M.E. (1997) Ras signalling linked to the cell-cycle machinery by the retinoblastoma protein [published erratum appears in Nature 1997 Apr 3;386(6624):521]. *Nature,* **386,** 177-81.

Pierce, A.M., Fisher, S.M., Conti, C.J. and Johnson, D.G. (1998) Deregulated expression of E2F1 induces hyperplasia and cooperates with ras in skin tumor development. *Oncogene,* **16,** 1267-76.

Polyak, K., Kato, J.Y., Solomon, M.J., Sherr, C.J., Massague, J., Roberts, J.M. and Koff, A. (1994) p27Kip1, a cyclin-Cdk inhibitor, links transforming growth factor-beta and contact inhibition to cell cycle arrest. *Genes Dev,* **8,** 9-22.

Schaap, D., van der Wal, J., Howe, L.R., Marshall, C.J. and van Blitterswijk, W.J. (1993) A dominant-negative mutant of raf blocks mitogen-activated protein kinase activation by growth factors and oncogenic p21ras. *J Biol Chem,* **268,** 20232-6.

Sears, R., Ohtani, K. and Nevins, J.R. (1997) Identification of positively and negatively acting elements regulating expression of the E2F2 gene in response to cell growth signals. *Mol Cell Biol,* **17,** 5227-35.

Serrano, M., Hannon, G.J. and Beach, D. (1993) A new regulatory motif in cell-cycle control causing specific inhibition of cyclin D/CDK4 [see comments]. *Nature,* **366,** 704-7.

Sherr, C.J. (1994) G1 phase progression: cycling on cue [see comments]. *Cell,* **79,** 551-5.

Singh, P., Wong, S.H. and Hong, W. (1994) Overexpression of E2F-1 in rat embryo fibroblasts leads to neoplastic transformation. *Embo J,* **13,** 3329-38.

Taniguchi, K., Kohsaka, H., Inoue, N., Terada, Y., Ito, H., Hirokawa, K. and Miyasaka, N. (1999) Induction of the p16INK4a senescence gene as a new therapeutic strategy for the treatment of rheumatoid arthritis [see comments]. *Nat Med,* **5,** 760-7.

Trouche, D., Cook, A. and Kouzarides, T. (1996) The CBP co-activator stimulates E2F1/DP1 activity. *Nucleic Acids Res,* **24,** 4139-45.

Trouche, D., Le Chalony, C., Muchardt, C., Yaniv, M. and Kouzarides, T. (1997) RB and hbrm cooperate to repress the activation functions of E2F1. *Proc Natl Acad Sci U S A,* **94,** 11268-73.

van der Eb, A.J. and Graham, F.L. (1980) Assay of transforming activity of tumor virus DNA. *Methods Enzymol,* **65,** 826-39.

Verona, R., Moberg, K., Estes, S., Starz, M., Vernon, J.P. and Lees, J.A. (1997) E2F activity is regulated by cell cycle-dependent changes in subcellular localization. *Mol Cell Biol,* **17,** 7268-82.

Wakino, S., Kintscher, U., Kim, S., Yin, F., Hsueh, W.A. and Law, R.E. (2000) Peroxisome proliferator-activated receptor gamma ligands inhibit retinoblastoma phosphorylation and G1--> S transition in vascular smooth muscle cells. *J Biol Chem,* **275,** 22435-41.

Weinberg, R.A. (1995) The retinoblastoma protein and cell cycle control. *Cell,* **81,** 323-30.

Xiao, Z.X., Ginsberg, D., Ewen, M. and Livingston, D.M. (1996) Regulation of the retinoblastoma protein-related protein p107 by G1 cyclin-associated kinases. *Proc Natl Acad Sci U S A,* **93,** 4633-7.

Xu, G., Livingston, D.M. and Krek, W. (1995) Multiple members of the E2F transcription factor family are the products of oncogenes. *Proc Natl Acad Sci U S A,* **92,** 1357-61.

Yamasaki, L., Jacks, T., Bronson, R., Goillot, E., Harlow, E. and Dyson, N.J. (1996) Tumor induction and tissue atrophy in mice lacking E2F-1. *Cell,* **85,** 537-48.

## Claims

**1.** A method for identifying a sample nucleic acid that modulates E2F activity, said method comprising:
a) introduction of sample nucleic acid into a reporter cell line containing at least 2 E2F binding sites operably linked to a heterologous reporter gene,
b) assaying the resulting reporter gene expression levels, wherein induction or reduction of expression of the heterologous reporter gene indicates that the sample nucleic acid is a modulator of E2F activity,
c) isolation of the sample nucleic acid that caused a modulation of expression of the reporter gene as measured in step b,
d) rescreening the nucleic acids of step c, said rescreening comprising the steps of: reintroducing said sample nucleic acid in the reporter cell line, and measurement of modulation of expression of said reporter gene,
e) validating those nucleic acids obtained from step d that caused a modulation of reporter gene expression, said validation comprising introduction of the nucleic acid into a cell line together with a transient E2F reporter and measurement of the fold induction or reduction of expression of said transient reporter gene.

**2.** A method according to claim 1 being **characterized in that** at least step a and b are performed in a miniaturized high-throughput format.

**3.** A method according to any of claims 1 or 2 wherein said sample nucleic acid of step c causes an induction of expression, said induction is at least the background signal obtained in the absence of a nucleic acid plus three times the standard deviation on the measurement of said background signal.

**4.** A method according to any of claims 1 or 2 wherein said sample nucleic acid of step c caused a repression of expression, said repression is at least a two-fold repression of reporter expression compared to said background reporter expression levels.

**5.** Any nucleic acid identifiable by the methods according to any of claims 1 to 4.

**6.** An isolated nucleic acid comprising a member selected from a group of nucleic acids identifiable as modulators of E2F reporter activity according to the method in any of claims 1 to 4, said group consisting of:
a) nucleic acid comprising a DNA sequence as given in SEQ ID NO 1, or 3. or the complement thereof,
b) nucleic acid comprising the RNA sequences corresponding to SEQ ID NO 1 or 3, or the complement thereof,
c) nucleic acid specifically hybridizing to the nucleotide sequence as defined in (a) or (b),
d) nucleic acid having a nucleotide sequence at least 65% identical to the sequence defined in (a),
e) nucleic acid encoding a protein with an amino acid sequence which is at least 65% identical to the amino acid sequence as given in SEQ ID NO 2 or 4,
f) nucleic acid encoding a protein comprising the amino acid sequence as given in any of SEQ ID NO 2 or 4,
g) nucleic acid which is degenerated as a result of the genetic code to a nucleotide sequence of a nucleic acid as given in SEQ ID NO 1 or 3, or as defined in (a) to (f),
h) nucleic acid which is diverging due to the differences in codon usage between the organisms to a nucleotide sequence encoding a protein as given in SEQ ID NO 2 or 4, or as defined in (a) to (g),
i) nucleic acid which is diverging due to the differences between alleles encoding a protein as given in SEQ ID NO 2 or 4, or as defined in (a) to (h),
j) nucleic acid encoding an immunologically active and/or functional fragment of a protein encoded by a DNA sequence as given in SEQ ID NO 1 or 3,
k) nucleic acid encoding a gene family member of the nucleic acid as given in SEQ ID NO 1 or 3, and,
l) nucleic acid encoding a protein as defined in SEQ ID NO 2 or 4 or a nucleic acid as defined in any one of (a) to (k) **characterized in that** said sequence is DNA, cDNA, genomic DNA or synthetic DNA.

**7.** A nucleic acid molecule of at least 15 nucleotides in length specifically hybridizing with a nucleic acid of claim 5 to 6.

**8.** A nucleic acid molecule of at least 15 nucleotides in length specifically amplifying a nucleic acid of claim 5 to 6.

**9.** A vector comprising a nucleic acid sequence according to claim 5 to 6.

**10.** A vector according to claim 9, wherein said vector is an expression vector wherein the nucleic acid sequence is operably linked to one or more control sequences allowing the expression of said sequence in prokaryotic and/or eukaryotic host cells.

**11.** A vector according to claim 9 or 10, wherein said vector is an adenoviral vector.

**12.** A vector according to claim 11, wherein said vector is generated from an adenoviral adapter vector which contains the left ITR and part of the E2B region, and in which the E1 region has been exchanged for a mammalian promotor, a polylinker sequence, and a polyadenylation signal.

**13.** A host cell containing an integrated or episomal copy of a nucleic acid molecule according to claim 5 to 6 or a vector according to claim 9 to 12.

**14.** The host cell of claim 13, wherein said host cell is a yeast, bacterial, insect, fungal, plant or mammalian cell.

**15.** An isolated polypeptide encodable by a nucleic acid of claim 5 to 6, or a variant or a derivative thereof, or an immunologically active and/or functional fragment thereof.

**16.** The polypeptide of claim 15 having an amino acid sequence as given in SEQ ID NO 2 or 4, or a variant or a derivative thereof, or an immunologically active and/or functional fragment thereof.

**17.** A method for producing a polypeptide according to claim 15 or 16 comprising culturing host cells of claim 13 or 14 under conditions allowing the expression of the polypeptide and recovering the produced polypeptide from the culture.

**18.** An antibody specifically recognizing a polypeptide of claim 15 or 16 or a specific epitope of said polypeptide.

**19.** A method for detecting a nucleic acid according to claim 5 to 6 or a polypeptide according to claim 15 or 16.

**20.** The use of a nucleic acid encoding a protein, comprising an amino acid sequence which is at least 65% identical to SEQ ID NO 2, for repressing E2F activity.

**21.** The use of a protein, comprising an amino acid sequence which is at least 65% identical to SEQ ID NO 2, for repressing E2F activity.

**22.** The use of a nucleic acid encoding a protein, comprising an amino acid sequence which is at least 65% identical to SEQ ID NO 4, for repressing E2F activity.

**23.** The use of a protein, comprising an amino acid sequence which is at least 65% identical to SEQ ID NO 4, for repressing E2F activity.

**24.** A nucleic acid, polypeptide or antibody according to any of the claims 1 to 18, for use as a medicament.

**25.** Use of a nucleic acid, polypeptide or antibody, according to any of the previous claims for the preparation of a medicament for preventing, treating and/or alleviating proliferative disorders and/or apoptosis-associated disorders.

**26.** Use of a nucleic acid, polypeptide or antibody, according to any of the previous claims for the preparation of a diagnostic kit for detecting proliferative disorders and/or apoptosis-associated disorders.

**27.** A method of diagnosing a pathological condition or a susceptibility to a pathological condition in a subject comprising the steps of:
a) determining the presence or absence of a mutation in the nucleic acid of claim 5 to 6, including mutations in the genomic and regulatory sequences of said nucleic acid, in a biological sample, and,
b) diagnosing a pathological condition or a susceptibility to a pathological condition based on the presence or absence of said mutation.

**28.** A method for diagnosing a pathological condition or a susceptibility to a pathological condition in a subject comprising the steps of:
a) determining the presence or amount of expression of the polypeptide of claim 15 or 16 or the nucleic acid of claim 5 to 6 in a biological sample, and,
b) diagnosing a pathological condition or a susceptibility to a pathological condition based on the presence or amount of expression of said polypeptide or said nucleic acid.

**29.** A method for screening compounds for preventing, treating or alleviating proliferative disorders or apoptosis-associated disorders comprising the steps of:
a) contacting the compounds to be screened with a nucleic acid according to any of claim 5 to 6, or with a polypeptide as defined in claim 15 or 16 , and,
b) determining whether said compound effects an activity of said nucleic acid or said polypeptide.

**30.** The product produced by the method of claim 29.

**31.** A method for the production of a composition comprising the steps of admixing a compound identifiable by a method of claim 29 with a pharmaceutically acceptable carrier.

**32.** A method for treating or alleviating proliferative disorders or apoptosis-associated disorders comprising the use of molecule which allows to interfere with the expression of a polypeptide as defined in claim 15 or 16 in a patient.

**33.** A kit for the diagnosis of proliferative disorders or apoptosis-associated disorders in a patient comprising a nucleic acid according to claim 5 to 6, a probe or primer according to claim 7 or 8, or an antibody according to claim 18.

**34.** A transgenic non-human animal comprising one or morecopies of a nucleic acid of claim 5 to 6 stably integrated in the genome, or an animal comprising regulatory elements that modulate the expression of a nucleic acid of claim 5 to 6.

**35.** A knock-out non-human animal comprising a deletion of one or two alleles encoding a nucleic acid of claim 5 to 6, or a animal comprising a targeted mutation in the genomic region, including regulatory sequences, comprising any of the nucleic acid sequences of claim 5 to 6.

**36.** Use of a transgenic or knock-out non-human animal according to claim 34 and 35 as a model system.

**37.** A gene therapy method for treating or alleviating proliferative disorders or apoptosis-associated disorders comprising the use of vectors according to claim 9 to 12.

**39.** A method for regulating cell proliferation or apoptosis, the method comprising introduction of a nucleic acid of claim 5 to 6 or an expression vector according to claim 9 to 12 in a desired target cell.

**40.** The use of a nucleic acid according to any of the previous claims for modulating E2F activity.


SEQUENCE LISTING

<110> GALAPAGOS GENOMICS BV

<120> High-throughput identification of modulators of disease
      pathways

<130> GAL-006-EP

<140>
<141>

<160> 4

<170> PatentIn Ver. 2.1

<210> 1
<211> 3181
<212> DNA
<213> HUMAN PLACENTA

<400> 1
gacggcaggg acacgcggaa ccaagacttg gaaacttgat tgttgtggtt cttcttgggg 60
gttatgaaat ttcattaatc tttttttttt ccggggagaa agtttttgga aagattcttc 120
cagatatttc ttcattttct tttggaggac cgacttactt tttttggtct tctttattac 180
tcccctcccc ccgtgggacc cgccggacgc gtggaggaga ccgtagctga agctgattct 240
gtacagcggg acagcgcttt ctgcccctgg gggagcaacc cctccctcgc ccctgggtcc 300
tacggagcct gcactttcaa gaggtacagc ggcatcctgt gggggcctgg gcaccgcagg 360
aagactgcac agaaactttg ccattgttgg aacgggacgt tgctccttcc ccgagcttcc 420
ccggacagcg tactttgagg actcgctcag ctcaccgggg actcccacgg ctcaccccgg 480
acttgcacct tacttcccca acccggccat agccttggct tcccggcgac ctcagcgtgg 540
tcacaggggc ccccctgtgc ccagggaaat gtttcaggct ttccccggag actacgactc 600
cggctcccgg tgcagctcct caccctctgc cgagtctcaa tatctgtctt cggtggactc 660
cttcggcagt ccacccaccg ccgcggcctc ccaggagtgc gccggtctcg ggaaatgccc 720
cggttccttc gtgcccacgg tcaccgcgat cacaaccagc caggacctcc agtggcttgt 780
gcaacccacc ctcatctctt ccatggccca gtcccagggg cagccactgg cctcccagcc 840
cccggtcgtc gacccctacg acatgccggg aaccagctac tccacaccag gcatgagtgg 900
ctacagcagt ggcggagcga gtggcagtgg tgggccttcc accagcggaa ctaccagtgg 960
gcctgggcct gcccgcccag cccgagcccg gcctaggaga ccccgagagg agacgctcac 1020
cccagaggaa gaggagaagc gaagggtgcg ccgggaacga aataaactag cagcagctaa 1080
atgcaggaac cggcggaggg agctgaccga ccgactccag gcggagacag atcagttgga 1140
ggaagaaaaa gcagagctgg agtcggagat cgccgagctc caaaaggaga aggaacgtct 1200
ggagtttgtg ctggtggccc acaaaccggg ctgcaagatc ccctacgaag aggggcccgg 1260
gccgggcccg ctggcggagg tgagagattt gccgggctca gcaccggcta aggaagatgg 1320
cttcagctgg ctgctgccgc ccccgccacc accgcccctg cccttccaga ccagccaaga 1380
cgcacccccc aacctgacgg cttctctctt tacacacagt gaagttcaag tcctcggcga 1440
ccccttcccc gttgttaacc cttcgtacac ttcttcgttt gtcctcacct gcccggaggt 1500
ctccgcgttc gccggcgccc aacgcaccag cggcagtgac cagccttccg atcccctgaa 1560

ctcgccctcc ctcctcgctc tgtgaactct ttagacacac aaaacaaaca aacacatggg 1620
ggagagagac ttggaagagg aggaggagga ggagaaggag gagagagagg ggaagagaca 1680
aagtgggtgt gtggcctccc tggctcctcc gtctgaccct ctgcggccac tgcgccactg 1740
ccatcggaca ggaggattcc ttgtgttttg tcctgcctct tgtttctgtg ccccggcgag 1800
gccggagagc tggtgacttt ggggacaggg ggtgggaagg ggatggacac ccccagctga 1860
ctgttggctc tctgacgtca acccaagctc tggggatggg tggggagggg ggcgggtgac 1920
gcccaccttc gggcagtcct gtgtgaggat taagggacgg gggtgggagg taggctgtgg 1980
ggtgggctgg agtcctctcc agagaggctc aacaaggaaa aatgccactc cctacccaat 2040
gtctcccaca cccacccttt ttttggggtg cctaggttgg tttcccctgc actcccgacc 2100
ttagcttatt gatcccacat ttccatggtg tgagatcctc tttactctgg gcagaagtga 2160
gccccccctt aaagggaatt cgatgccccc ctagaataat ctcatccccc cacccgactt 2220
cttttgaaat gtgaacgtcc ttccttgact gtctagccac tccctcccag aaaaactggc 2280
tctgattgga atttctggcc tcctaaggct ccccaccccg aaatcagccc ccagccttgt 2340
ttctgatgac agtgttatcc caagaccctg ccccctgcca gccgaccctc ctggccttcc 2400
tcgttgggcc gctctgattt caggcagcag gggctgctgt gatgccgtcc tgctggagtg 2460
atttatactg tgaaatgagt tggccagatt gtggggtgca gctgggtggg gcagcacacc 2520
tctgggggga taatgtcccc actcccgaaa gcctttcctc ggtctccctt ccgtccatcc 2580
cccttcttcc tcccctcaac agtgagttag actcaagggg gtgacagaac cgagaagggg 2640
gtgacagtcc tccatccacg tggcctctct ctctctcctc aggaccctca gccctggcct 2700
ttttctttaa ggtcccccga ccaatcccca gcctaggacg ccaacttctc ccaccccttg 2760
gcccctcaca tcctctccag gaagggagtg aggggctgtg acatttttcc ggagaagatt 2820
tcagagctga ggctttggta cccccaaacc cccaatattt ttggactggc agactcaagg 2880
ggctggaatc tcatgattcc atgcccgagt ccgcccatcc ctgaccatgg ttttggctct 2940
cccaccccgc cgttccctgc gcttcatctc atgaggattt ctttatgagg caaatttata 3000
ttttttaata tcggggggtg gaccacgccg ccctccatcc gtgctgcatg aaaaacattc 3060
cacgtgcccc ttgtcgcgcg tctcccatcc tgatcccaga cccattcctt agctatttat 3120
ccctttcctg gtttccgaaa ggcaattata tctattatgt ataagtaaaa aaaaaaaaaa 3180
a 3181

<210> 2
<211> 338
<212> PRT
<213> HUMAN PLACENTA

<400> 2
Met Phe Gln Ala Phe Pro Gly Asp Tyr Asp Ser Gly Ser Arg Cys Ser
1               5                   10                  15

Ser Ser Pro Ser Ala Glu Ser Gln Tyr Leu Ser Ser Val Asp Ser Phe
            20                  25                  30

Gly Ser Pro Pro Thr Ala Ala Ala Ser Gln Glu Cys Ala Gly Leu Gly
        35                  40                  45

Glu Met Pro Gly Ser Phe Val Pro Thr Val Thr Ala Ile Thr Thr Ser
    50                  55                  60

Gln Asp Leu Gln Trp Leu Val Gln Pro Thr Leu Ile Ser Ser Met Ala
65                  70                  75                  80

Gln Ser Gln Gly Gln Pro Leu Ala Ser Gln Pro Pro Val Val Asp Pro
                85                  90                  95

Tyr Asp Met Pro Gly Thr Ser Tyr Ser Thr Pro Gly Met Ser Gly Tyr
            100                 105                 110

Ser Ser Gly Gly Ala Ser Gly Ser Gly Gly Pro Ser Thr Ser Gly Thr
        115                 120                 125

Thr Ser Gly Pro Gly Pro Ala Arg Pro Ala Arg Ala Arg Pro Arg Arg
    130                 135                 140

Pro Arg Glu Glu Thr Leu Thr Pro Glu Glu Glu Glu Lys Arg Arg Val
145                 150                 155                 160

Arg Arg Glu Arg Asn Lys Leu Ala Ala Ala Lys Cys Arg Asn Arg Arg
                165                 170                 175

Arg Glu Leu Thr Asp Arg Leu Gln Ala Glu Thr Asp Gln Leu Glu Glu
            180                 185                 190

Glu Lys Ala Glu Leu Glu Ser Glu Ile Ala Glu Leu Gln Lys Glu Lys
        195                 200                 205

Glu Arg Leu Glu Phe Val Leu Val Ala His Lys Pro Gly Cys Lys Ile
    210                 215                 220

Pro Tyr Glu Glu Gly Pro Gly Pro Gly Pro Leu Ala Glu Val Arg Asp
225                 230                 235                 240

Leu Pro Gly Ser Ala Pro Ala Lys Glu Asp Gly Phe Ser Trp Leu Leu
                245                 250                 255

Pro Pro Pro Pro Pro Pro Pro Leu Pro Phe Gln Thr Ser Gln Asp Ala
            260                 265                 270

Pro Pro Asn Leu Thr Ala Ser Leu Phe Thr His Ser Glu Val Gln Val
        275                 280                 285

Leu Gly Asp Pro Phe Pro Val Val Asn Pro Ser Tyr Thr Ser Ser Phe
    290                 295                 300

Val Leu Thr Cys Pro Glu Val Ser Ala Phe Ala Gly Ala Gln Arg Thr
305                 310                 315                 320

Ser Gly Ser Asp Gln Pro Ser Asp Pro Leu Asn Ser Pro Ser Leu Leu
                325                 330                 335

Ala Leu

<210> 3
<211> 974
<212> DNA
<213> HUMAN PLACENTA

<400> 3
ggctcgcccg ctttcgcacc cagttcacgc gccacagcta tgtgtccccg agccgcgcgg 60
gcgcccgcga cgctactcct cgccctgggc gcggtgctgt ggcctgcggc tggcgcctgg 120
gagcttacga ttttgcacac caacgacgtg cacagccggc tggagcagac cagcgaggac 180
tccagcaagt gcgtcaacgc cagccgctgc atgggtggcg tggctcggct cttcaccaag 240
gttcagcaga tccgccgcgc cgaacccaac gtgctgctgc tggacgccgg cgaccagtac 300
cagggcacta tctggttcac cgtgtacaag ggcgccgagg tggcgcactt catgaacgcc 360
ctgcgctacg atgccatggc actgggaaat catgaatttg ataatggtgt ggaaggactg 420
atcgagccac tcctcaaaga ggccaaattt ccaattctga gtgcaaacat taaagcaaag 480
gggccactag catctcaaat atcaggactt tatttgccat ataaagttct tcctgttggt 540
gatgaagttg tgggaatcgt tggatacact tccaaagaaa ccccttttct ctcaaatcca 600
gggacaaatt tagtgtttga agatgaaatc actgcattac aacctgaagt agataagtta 660
aaaactctaa atgtgaacaa aattattgca ctgggacatt cgggttttga aatggataaa 720
ctcatcgctc agaaagtgag gggtgtggac gtcgtggtgg gaggacactc caacacattt 780
ctttacacag gtaattgttt caaaaggatt gcatggccca ggatgtccag ataagcactg 840
tgtctctttt gcctttgtaa ctgttattac tctttttact gctatttaat atgtaatgta 900
tattatatga tctataatat atatgtaata tacattaaat gggaacatgt gcaaatctta 960
aaaaaaaaaa aaaa                                                   974

<210> 4
<211> 264
<212> PRT
<213> HUMAN PLACENTA

<400> 4
Met Cys Pro Arg Ala Ala Arg Ala Pro Ala Thr Leu Leu Leu Ala Leu
1               5                   10                  15

Gly Ala Val Leu Trp Pro Ala Ala Gly Ala Trp Glu Leu Thr Ile Leu
            20                  25                  30

His Thr Asn Asp Val His Ser Arg Leu Glu Gln Thr Ser Glu Asp Ser
        35                  40                  45

Ser Lys Cys Val Asn Ala Ser Arg Cys Met Gly Gly Val Ala Arg Leu
    50                  55                  60

Phe Thr Lys Val Gln Gln Ile Arg Arg Ala Glu Pro Asn Val Leu Leu
65                  70                  75                  80

Leu Asp Ala Gly Asp Gln Tyr Gln Gly Thr Ile Trp Phe Thr Val Tyr
                85                  90                  95

Lys Gly Ala Glu Val Ala His Phe Met Asn Ala Leu Arg Tyr Asp Ala
            100                 105                 110

Met Ala Leu Gly Asn His Glu Phe Asp Asn Gly Val Glu Gly Leu Ile
        115                 120                 125

Glu Pro Leu Leu Lys Glu Ala Lys Phe Pro Ile Leu Ser Ala Asn Ile
    130                 135                 140

Lys Ala Lys Gly Pro Leu Ala Ser Gln Ile Ser Gly Leu Tyr Leu Pro
145                 150                 155                 160

Tyr Lys Val Leu Pro Val Gly Asp Glu Val Val Gly Ile Val Gly Tyr
                165                 170                 175

Thr Ser Lys Glu Thr Pro Phe Leu Ser Asn Pro Gly Thr Asn Leu Val
            180                 185                 190

Phe Glu Asp Glu Ile Thr Ala Leu Gln Pro Glu Val Asp Lys Leu Lys
        195                 200                 205

Thr Leu Asn Val Asn Lys Ile Ile Ala Leu Gly His Ser Gly Phe Glu
    210                 215                 220

Met Asp Lys Leu Ile Ala Gln Lys Val Arg Gly Val Asp Val Val Val
225                 230                 235                 240

Gly Gly His Ser Asn Thr Phe Leu Tyr Thr Gly Asn Cys Phe Lys Arg
                245                 250                 255

Ile Ala Trp Ala Arg Met Ser Arg
            260